(19) Europäisches Patentamt
European Patent Office
Office européen des brevets

(11) Veröffentlichungsnummer: **0 260 228 A2**

(12) **EUROPÄISCHE PATENTANMELDUNG**

(21) Anmeldenummer: **87810515.4**

(22) Anmeldetag: **07.09.87**

(51) Int. Cl.4: **C 07 D 207/448**
C 07 D 207/404,
A 01 N 43/36, C 07 D 405/10

(30) Priorität: **12.09.86 CH 3664/86**
**28.04.87 CH 1616/87**

(43) Veröffentlichungstag der Anmeldung:
**16.03.88 Patentblatt 88/11**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Moser, Hans, Dr.**
**Maispracherstrasse 12**
**CH-4312 Magden (CH)**

**Pissiotas, Georg, Dr.**
**Am Sonnenrain 71**
**D-7850 Lörrach (DE)**

**Brunner, Hans-Georg, Dr.**
**Wannenstrasse 14**
**CH-4415 Lausen (CH)**

**Böhner, Beat, Dr.**
**Hügelweg 3**
**CH-4102 Binningen (CH)**

**Baumann, Marcus, Dr.**
**Hammerstrasse 82**
**CH-4057 Basel (CH)**

# (54) N-Phenyl-maleinimide und N-Phenyl-succinimide mit herbizider und pflanzenwuchsregulierender Wirkung.

(57) Neue Derivate des N-Phenylmaleinsäureimides und des N-Phenylsuccinimides der untenstehenden Formel I haben gute pre- und postemergente selektiv-herbizide Eigenschaften und sie beeinflussen das Pflanzenwachstum. Die Verbindungen entsprechen der Formel I

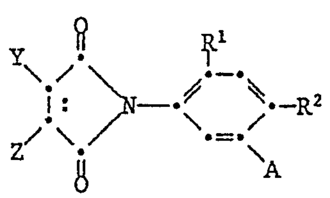

(I)

worin
|: für eine Einfach- oder Doppelbindung,
$R^1$ für Wasserstoff und Fluor,
$R^2$ für Halogen,
Y für $C_1$-$C_8$-Alkyl,
Z für Wasserstoff oder $C_1$-$C_8$-Alkyl, und
A für Wasserstoff oder eine Gruppe

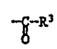, 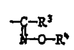, 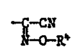, 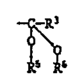, 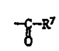 oder $-X-R^8$ stehen,

stehen,
wobei
$R^3$, $R^4$, $R^5$, $R^6$, $R^7$ und $R^8$ übliche organische Reste und X Sauerstoff, Schwefel, SO, $SO_2$, NH, Alkylimino oder Alkenylimino bedeuten.

EP 0 260 228 A2

**Beschreibung**

N-Phenyl-maleinimide und N-Phenyl-succinimide mit herbizider und pflanzenwuchsregulierender Wirkung

Die vorliegende Erfindung betrifft neue Derivate des N-Phenylmaleinimids und N-Phenyl-succinimids mit herbizider und pflanzenwuchsregulierender Wirkung, agrochemische Mittel, welche diese Substanzen als Wirkstoffe enthalten, die Verwendung der neuen N-Phenyltetrahydrophthalimide zur selektiven Bekämpfung von Unkräutern oder zur Regulierung von Pflanzenwuchs sowie Verfahren zur Herstellung dieser neuen Verbindungen. Ferner betrifft die Erfindung auch neue Zwischenprodukte, welche zur Herstellung der neuen Wirkstoffe dienen.

Die neuen Derivate des N-Phenylmaleinimids und N-Phenylsuccinimids entsprechen der Formel I

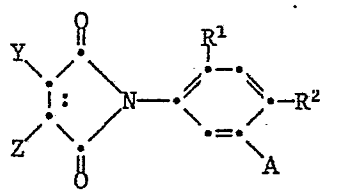

(I)

worin |: für eine Einfach- oder Doppelbindung,
$R^1$ für Wasserstoff oder Fluor,
$R^2$ für Halogen,
Y für $C_1$-$C_8$-Alkyl,
Z für Wasserstoff oder $C_1$-$C_8$-Alkyl und
A für Wasserstoff oder eine Gruppe

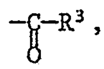 , 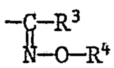 , 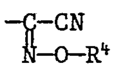 , 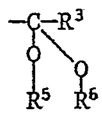 , 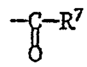 oder $-X-R^8$ stehen,

wobei
$R^3$ Wasserstoff, $C_1$-$C_4$-Alkyl oder $C_1$-$C_4$-Halogenalkyl,
$R^4$ Wasserstoff, $C_1$-$C_8$-Alkyl, $C_2$-$C_8$-Alkoxyalkyl, $C_2$-$C_8$-Alkylthioalkyl, $C_2$-$C_8$-Alkylaminoalkyl, $C_1$-$C_8$-Halogenalkyl, $C_1$-$C_8$-Cyanoalkyl, $C_3$-$C_8$-Alkenyl, $C_3$-$C_8$-Halogenalkenyl, $C_3$-$C_8$-Alkinyl, $C_3$-$C_7$-Cycloalkyl, $C_3$-$C_7$-Halogencycloalkyl, $C_1$-$C_4$-Alkylsulfonyl, ein Natriumion, ein Kaliumion, ein Magnesiumion, ein Calciumion, ein Ammoniumion, $C_1$-$C_4$-Alkoxycarbonyl, Benzyloxycarbonyl, $C_5$-$C_7$-Cycloalkoxycarbonyl, Phenoxycarbonyl, das unsubstituiert oder am Phenylring einfach durch Halogen, $C_1$-$C_4$-Alkyl, $C_1$-$C_4$-Alkoxy, $C_1$-$C_4$-Halogenalkyl, Cyan oder Nitro substituiert ist, $C_1$-$C_4$-Alkylthiocarbonyl, $C_1$-$C_4$-Alkylaminocarbonyl, Dimethylaminocarbonyl, Benzylaminocarbonyl, $C_3$-$C_7$-Cycloalkylaminocarbonyl, Phenylaminocarbonyl, das unsubstituiert oder am Phenylkern einfach durch Halogen, $C_1$-$C_4$-Alkyl, $C_1$-$C_4$-Alkoxy, $C_1$-$C_4$-Halogenalkyl, Cyan oder Nitro substituiert ist, $C_1$-$C_8$-Alkylcarbonyl, Allylcarbonyl, Benzylcarbonyl, das unsubstituiert oder am Phenylring einfach durch Halogen, $C_1$-$C_4$-Alkyl, $C_1$-$C_4$-Alkoxy, $C_1$-$C_4$-Halogenalkyl, Cyan oder Nitro substituiert ist, $C_3$-$C_7$-Cycloalkylcarbonyl, Benzoyl, das unsubstituiert oder am Phenylring einfach durch Halogen, $C_1$-$C_4$-Alkyl, $C_1$-$C_4$-Alkoxy, $C_1$-$C_4$-Halogenalkyl, Cyan oder Nitro substituiert ist, Furoyl, Thenoyl; oder $C_1$-$C_4$-Alkyl substituiert durch Phenyl, Halogenphenyl, $C_1$-$C_4$-Alkylphenyl, $C_1$-$C_4$-Alkoxyphenyl, $C_1$-$C_4$-Haloalkylphenyl, $C_1$-$C_4$-Halogenalkoxyphenyl, Nitrophenyl, Cyanophenyl, $C_1$-$C_8$-Alkoxycarbonyl, $C_2$-$C_8$-Alkoxyalkoxycarbonyl, $C_3$-$C_8$-Alkenyloxycarbonyl, $C_3$-$C_8$-Alkinyloxycarbonyl, $C_1$-$C_8$-Alkylthiocarbonyl, $C_3$-$C_8$-Alkenylthiocarbonyl, $C_3$-$C_8$-Alkinylthiocarbonyl, Carbamoyl, $C_1$-$C_4$-Alkylaminocarbonyl, Di-$C_1$-$C_4$-alkylaminocarbonyl, Phenylaminocarbonyl, das unsubstituiert oder am Phenylring einfach durch Halogen, $C_1$-$C_4$-Alkyl, $C_1$-$C_4$-Alkoxy, Cyan, Nitro oder $C_1$-$C_4$-Halogenalkyl substituiert ist, Dioxolan-2-yl, das unsubstituiert oder durch ein bis zwei $C_1$-$C_4$-Alkylreste substituiert ist, oder 1,3-Dioxan-2-yl, das unsubstituiert oder durch ein bis zwei $C_1$-$C_4$-Alkylreste substituiert ist;
$R^5$ und $R^6$ unabhängig voneinander jeweils $C_1$-$C_4$-Alkyl, $C_2$-$C_4$-Halogenalkyl oder $C_2$-$C_8$-Alkoxyalkyl, oder
$R^5$ und $R^6$ zusammen eine Aethylen- oder Propylenbrücke oder einen 1,2-Cyclohexanylenkörper, wobei diese Gruppen unsubstituiert sind oder durch ein bis zwei Reste aus der Gruppe $C_1$-$C_4$-Alkyl, $C_1$-$C_4$-Halogenalkyl oder $C_1$-$C_4$-Hydroxyalkyl substituiert sind,
$R^7$ Hydroxy, $C_1$-$C_8$-Alkoxy, $C_2$-$C_8$-Alkoxyalkoxy, $C_2$-$C_8$-Alkylthioalkoxy, $C_2$-$C_8$-Alkylaminoalkoxy, $C_1$-$C_8$-Cyanoalkoxy, $C_3$-$C_8$-Alkenyloxy, $C_2$-$C_8$-Halogenalkenyloxy, $C_3$-$C_8$-Alkinyloxy, $C_3$-$C_7$-Cycloalkoxy, $C_3$-$C_7$-Halogencycloalkoxy, Benzloxy das unsubstituiert oder am Phenylkern einfach durch Halogen, $C_1$-$C_4$-Alkyl, $C_1$-$C_4$-Alkoxy, $C_1$-$C_4$-Halogenalkyl, Cyan oder Nitro substituiert ist, Phenoxy, Halogenphenoxy, $C_1$-$C_4$-Alkylphenoxy, $C_1$-$C_4$-Alkoxyphenoxy, $C_1$-$C_4$-Halogenalkylphenoxy, Cyanophenoxy, Nitrophenoxy, Phenylthio, Halogenphenylthio, $C_1$-$C_4$-Alkylphenylthio, $C_1$-$C_4$-Alkoxyphenylthio, $C_1$-$C_4$-Halogenalkylthio, Cyanophenylthio, Nitrophenylthio, die Salzgruppen -O-Na, -O-K, -O-Ca, -O-Mg, -O-$NH_4$; Amino, $C_1$-$C_4$-Alkylamino, Di-$C_1$-$C_4$-alkylamino, $C_2$-$C_4$-Halogenalkylamino, Di-$C_2$-$C_4$-halogenalkylamino, $C_1$-$C_4$-Hydroxyalkylamino, Di-

$C_1$-$C_4$-hydroxyalkylamino, $C_3$-$C_4$-Alkenylamino, Diallylamino, -N-Pyrrolidino, -N-Piperidino, -N-Morpholino, -N-Thiomorpholino, -N-Piperidazino, $C_1$-$C_8$-Alkylthio, $C_3$-$C_8$-Alkenylthio, Benzylthio, $C_1$-$C_4$-Alkylthio substituiert durch $C_1$-$C_8$-Alkoxycarbonyl, $C_2$-$C_8$-Alkoxyalkoxycarbonyl, $C_3$-$C_8$-Alkenyloxycarbonyl, $C_3$-$C_8$-Alkinyloxycarbonyl, $C_1$-$C_8$-Alkylthiocarbonyl, $C_3$-$C_8$-Alkenylthiocarbonyl oder $C_3$-$C_8$-Alkinylthiocarbonyl; oder $C_1$-$C_4$-Alkoxy substituiert durch $C_1$-$C_8$-Alkoxycarbonyl, $C_2$-$C_8$-Alkoxyalkoxycarbonyl, $C_3$-$C_8$-Alkenyloxycarbonyl, $C_3$-$C_8$-Alkinyloxycarbonyl, $C_1$-$C_8$-Alkylthiocarbonyl, $C_3$-$C_8$-Alkenylthiocarbonyl, $C_3$-$C_8$-Alkinylthiocarbonyl, $C_1$-$C_4$-Alkylaminocarbonyl, Di-$C_1$-$C_4$-alkylaminocarbonyl oder Phenylaminocarbonyl das unsubstituiert oder am Phenylkern einfach durch Halogen, $C_1$-$C_4$-Alkyl, $C_1$-$C_4$-Alkoxy, $C_1$-$C_4$-Halogenalkyl, Cyan oder Nitro substituiert ist;

X Sauerstoff, Schwefel, -SO-, $-SO_2-$, -NH-, -N($C_1$-$C_4$-Alkyl)- oder -N($C_3$-$C_4$-Alkenyl)-, und

und

$R^8$ $C_1$-$C_8$-Alkyl, $C_2$-$C_8$-Alkoxyalkyl, $C_2$-$C_8$-Alkylthioalkyl, $C_2$-$C_8$-Alkylaminoalkyl, $C_2$-$C_8$-Halogenalkyl, $C_1$-$C_8$-Cyanoalkyl, $C_3$-$C_8$-Alkenyl, $C_2$-$C_8$-Halogenalkenyl, $C_3$-$C_8$-Alkinyl, $C_3$-$C_7$-Cycloalkyl, $C_3$-$C_7$-Halogencycloalkyl, $C_1$-$C_8$-Alkylcarbonyl, Allylcarbonyl, Benzylcarbonyl, das unsubstituiert oder am Phenylring einfach durch Halogen, $C_1$-$C_4$-Alkyl, $C_1$-$C_4$-Alkoxy, $C_1$-$C_4$-Halogenalkyl, Cyan oder Nitro substituiert ist, $C_3$-$C_7$-Cycloalkylcarbonyl, Benzoyl, das unsubstituiert oder am Phenylring einfach durch Halogen, $C_1$-$C_4$-Alkyl, $C_1$-$C_4$-Alkoxy, $C_1$-$C_4$-Halogenalkyl, Cyan oder Nitro substituiert ist, Furoyl, Thienoyl oder $C_1$-$C_4$-Alkyl substituiert durch Phenyl, Halogenphenyl, $C_1$-$C_4$-Alkylphenyl, $C_1$-$C_4$-Alkoxyphenyl, $C_1$-$C_4$-Haloalkylphenyl, $C_1$-$C_4$-Halogenalkoxyphenyl, Nitrophenyl, Cyanophenyl, $C_1$-$C_8$-Alkoxycarbonyl, $C_2$-$C_8$-Alkoxyalkoxycarbonyl, $C_3$-$C_8$-Alkenyloxycarbonyl, $C_3$-$C_8$-Alkinyloxycarbonyl, $C_1$-$C_8$-Alkylthiocarbonyl, $C_3$-$C_8$-Alkenylthiocarbonyl, $C_3$-$C_8$-Alkinylthiocarbonyl, Carbamoyl, $C_1$-$C_4$-Alkylaminocarbonyl, Di-$C_1$-$C_4$-alkylaminocarbonyl, Phenylaminocarbonyl, das unsubstitiert oder am Phenylring einfach durch Halogen, $C_1$-$C_4$-Alkyl, $C_1$-$C_4$-Alkoxy, Cyan, Nitro oder $C_1$-$C_4$-Halogenalkyl substituiert ist; Dioxolan-2-yl, das unsubstituiert oder durch ein bis zwei $C_1$-$C_4$-Alkylreste substituiert ist, oder 1,3-Dioxan-2-yl, das unsubstituiert oder durch ein bis zwei $C_1$-$C_4$-Alkylreste substituiert ist, bedeuten.

Aus der Literatur sind Derivate der N-Phenyl-2,3-dimethylmaleinsäureimide als Herbizide aus publizierten Patentanmeldungen DE-A 2 735 841 oder JA-A 57.144 204 sowie aus dem US Patent 4 138 243 bekannt geworden.

In der obigen Definition umfassen die angegebenen generischen Begriffe beispielsweise die folgenden spezifischen Einzelsubstituenten, wobei diese Aufzählung keine Einschränkung der Erfindung darstellt:

Alkyl: Methyl, Aethyl, n-Propyl, i-Propyl, n-Butyl, i-Butyl, s-Butyl und t-Butyl, vorzugsweise Methyl und Aethyl.

Halogen: Fluor, Chlor, Brom und Jod, vorzugsweise Fluor, Chlor und Brom.

Alkoxy: Methoxy, Aethoxy, Propyloxy, i-Propyloxy, n-Butyloxy, i-Butyloxy, s-Butyloxy und t-Butyloxy, vorzugsweise Methoxy oder Aethoxy.

Halogenalkyl: Fluormethyl, Difluormethyl, Trifluormethyl, Chlormethyl, Dichlormethyl, Trichlormethyl, 2,2,2-Trifluoräthyl, 2-Fluoräthyl, 2-Chloräthyl und 2,2,2-Trichloräthyl, vorzugsweise Chlormethyl, 2-Chloräthyl und Trifluormethyl.

Halogenalkoxy: Fluormethoxy, Difluormethoxy, Trifluormethoxy, 2,2,2-Trifluoräthoxy, 1,1,2,2-Tetrafluoräthoxy, 2-Fluoräthoxy, 2-Chloräthoxy und 2,2,2-Trichloräthoxy, vorzugsweise Difluormethoxy, 2-Chloräthoxy und Trifluormethoxy.

Alkoxycarbonyl: Methoxycarbonyl, Aethoxycarbonyl, 4-Propyloxycarbonyl, i-Propyloxycarbonyl und n-Butyloxycarbonyl, vorzugsweise Methoxycarbonyl und Aethoxycarbonyl.

Alkoxyalkyl: Methoxymethyl, Aethoxymethyl, Propyloxymethyl, Methoxyäthyl, Aethoxyäthyl, Propyloxyäthyl, Methoxypropyl, Aethoxypropyl oder Propyloxypropyl.

Alkylthioalkyl: Methylthiomethyl,Aethylthiomethyl, Methylthioäthyl, Aethylthioäthyl, oder Isopropylthioäthyl.

Alkylaminoalkyl: Methylaminoäthyl, Dimethylaminoäthyl, Aethylaminoäthyl oder Diäthylaminoäthyl.

Cyanoalkyl: Cyanomethyl, Cyanoäthyl oder Cyanopropyl.

Alkenyl: Allyl, 2-Butenyl, 3-Butenyl oder Methallyl, vorzugsweise aber Allyl.

Alkinyl: Propargyl, 2-Butinyl oder 3-Butinyl, vorzugsweise aber Propargyl.

Cycloalkyl: Cyclopropyl, Cyclobutyl, Cyclopentyl, Cyclohexyl oder Cycloheptyl. Bevorzugt sind Cyclopentyl oder Cyclohexyl.

Halogencycloalkyl: 2,2-Dichlorcyclopropyl oder Pentachlorcyclohexyl.

Alkylsulfonyl: Methylsulfonyl, Aethylsulfonyl, Propylsulfonyl oder Butylsulfonyl. Bevorzugt sind Methyl- und Aethylsulfonyl.

Cycloalkoxycarbonyl: Cyclopentyloxycarbonyl oder Cyclohexyloxycarbonyl.

Phenyl, auch als Teil eines grösseren Substituenten wie Phenoxy, Phenylthio, Phenoxycarbonyl, Phenylaminocarbonyl, Benzyl oder Benzoyl, kann im allgemeinen unsubstituiert oder durch einen weiteren Substituenten substituiert vorliegen. Die Substituenten können dann in ortho-, meta- oder para-Stellung stehen. Bevorzugte Substituentenstellungen sind die ortho- und para-Position zur Ringverknüpfungsstelle. Bevorzugte Substituenten sind Halogenatome.

In den weiteren Substituenten, welche aus mehreren Grundelementen zusammengesetzt sind, haben die Teilelemente die oben an Beispielen erläuterten Bedeutungen. Diese Aufzählungen bedeuten auch in diesen Fällen keine Einschränkdung der Erfindung: sie haben illustrierenden Charakter.

Von den erfindungsgemässen Verbindungen sind diejenigen bevorzugt, in denen entweder

a) die Imide der Formel I, in denen

$R^1$ Wasserstoff oder Fluor,
$R^2$ Chlor oder Brom,
Y $C_1$-$C_8$-Alkyl,
Z Wasserstoff oder $C_1$-$C_8$-Alkyl,
A den Rest -$COR^3$ und
$R^3$ Wasserstoff, $C_1$-$C_4$-Alkyl oder $C_1$-$C_4$-Halogenalkyl bedeuten;

b) die Imide der Formel I, in denen
$R^1$ Wasserstoff oder Fluor,
$R^2$ Chlor oder Brom,
Y $C_1$-$C_8$-Alkyl,
Z Wasserstoff oder $C_1$-$C_8$-Alkyl
A Den Rest -$CR^3=NOR^4$ und
$R^3$ und $R^4$ die unter der Formel I gegebene Bedeutung haben;

c) die Imide der Formel I, in denen
$R^1$ Wasserstoff oder Fluor,
$R^2$ Chlor oder Brom,
Y $C_1$-$C_8$-Alkyl,
Z Wasserstoff oder $C_1$-$C_8$-Alkyl,
A den Rest -$C(CN)=NOR^4$ bedeuten, und
$R^4$ die unter der Formel I gegebene Bedeutung hat;

d) die Imide der Formel I, in denen
$R^1$ Wasserstoff oder Fluor,
$R^2$ Chlor oder Brom,
Y $C_1$-$C_8$-Alkyl,
Z Wasserstoff oder $C_1$-$C_8$-Alkyl,
A den Rest

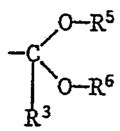

bedeuten und
$R^3$, $R^5$ und $R^6$ die unter der Formel I gegebene Bedeutung haben;

e) die Imide der Formel I, in denen
$R^1$ Wasserstoff oder Fluor,
$R^2$ Chlor oder Brom,
Y $C_1$-$C_8$-Alkyl,
Z Wasserstoff oder $C_1$-$C_8$-Alkyl,
A den Rest -$COR^7$ bedeuten, und
$R^7$ die unter der Formel I gegebene Bedeutung hat;

f) die Imide der Formel I, in denen
$R^1$ Wasserstoff oder Fluor,
$R^2$ Chlor oder Brom,
Y $C_1$-$C_8$-Alkyl,
Z Wasserstoff oder $C_1$-$C_8$-Alkyl,
A den Rest -$XR^8$ bedeuten, und
X und $R^8$ die unter der Formel I gegebene Bedeutung haben,
speziell
N-(2-Fluor-4-chlor-5-isopropoxyphenyl)-2,3-dimethyl-maleinsäureimid,
N-(2-Fluor-4-chlor-5-isopropoxyphenyl)-2-n-butyl-3-methyl-maleinsäureimid und
N-(2-Fluor-4-chlor-5-isopropoxyphenyl)-2-ethyl-3-methylmaleinsäureimid;

g) die Imide der Formel I, in denen
$R^1$ Wasserstoff oder Fluor,
$R^2$ Chlor oder Brom,
Y und Z je Methyl bedeuten, und
A eine der unter der Formel I gegebenen Bedeutungen hat;

h) die Imide der Formel I, in denen
$R^1$ Wasserstoff oder Fluor,
$R^2$ Chlor oder Brom,
Y Aethyl,
Z Methyl bedeuten, und
A eine der unter der Formel I gegebenen Bedeutungen hat;

i) die Imide der Formel I, in denen

$R^1$ Wasserstoff oder Fluor,
$R^2$ Chlor oder Brom,
Y Isopropyl,
Z Methyl bedeuten und
A eine der unter der Formel I gegebenen Bedeutungen hat;
k) die Imide der Formel I, in denen
$R^1$ Wasserstoff oder Fluor,
$R^2$ Chlor oder Brom,
Y Isopropyl,
Z Wasserstoff bedeuten, und
A eine der unter der Formel I gegebenen Bedeutungen hat;
l) die Imide der Formel I, in denen
$R^1$ Wasserstoff oder Fluor,
$R^2$ Chlor oder Brom,
Y n-Butyl,
Z Methyl bedeuten, und
A eine der unter der Formel I gegebenen Bedeutungen hat.

Die Verbindungen der Formel I werden erfindungsgemäss hergestellt, indem man ein Maleinsäureanhydrid oder ein Bernsteinsäureanhydrid der Formel II

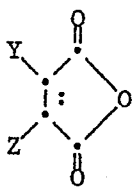

(II)

worin |: für eine Einfach- oder Doppelbindung steht und Y und Z die unter der Formel I gegebene Bedeutung haben, mit einem Anilin der Formel III

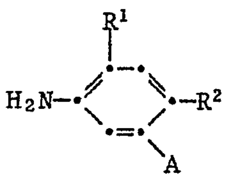

(III)

worin A, $R^1$ und $R^2$ die unter Formel I gegebenen Bedeutungen haben, kondensiert.

Mit Vorteil führt man die obige Kondensationsreaktion in einem inerten organischen Lösungsmittel aus. Die Reaktionstemperatur liegt im allgemeinen zwischen der Raumtemperatur und der Siedetemperatur des Reaktionsgemisches, vorzugsweise wird die Reaktionsmischung zum Rückfluss erhitzt. Die Kondensationsreaktion kann durch Zusatz von Kondensationskatalysatoren und Entfernung des entstehenden Reaktionsproduktes Wasser beschleunigt werden. Eine gleiche Wirkung erzielt man durch Zusatz von wasserentziehenden Mitteln, wie beispielsweise Schwefelsäure.

Als Lösungsmittel eignen sich insbesondere höhersiedende Kohlenwasserstoffe, niedere Alkancarbonsäuren sowie deren Ester und Amide, höhersiedende Ketone und Aether. Beispiele dafür sind Benzol, Toluol, Xylol, Dimethylformamid, Dimethylacetamid, Essigsäure, Aethylacetat, Diisopropyläther, Aethylenglykoldimethyläther, Tetrahydrofuran, Dioxan oder 2-Butanon.

Als Katalysatoren kommen insbesondere dann in Betracht, wenn ein unprotisches Lösungsmittel verwendet wird: p-Toluolsulfonsäure, Benzoesäure, 4-Dimethylaminopyridin, Schwefelsäure, Chlorwasserstoff oder Naphthalinsulfonsäure. Reaktionsführungen gemäss dem oben genannten Typ werden bei der Darstellung von Carbonsäure-Derivaten üblicherweise angewendet. Sie entsprechen der allgemein üblichen Laboratoriumspraxis.

Die Anhydride der Formel II sind bekannt und können nach analog zu bekannten Verfahren hergestellt werden.

Die Anilin-Derivate der Formel III sind neu und wurden speziell für die Synthese der Verbindungen der Formel I entwickelt und hergestellt. Sie bilden daher einen Gegenstand der vorliegenden Erfindung.

Die Darstellung der Verbindungen der Formel III erfolgt durch sinngemässe Aneinanderreihung von an sich bekannten Reaktionsschritten wie Verätherung einer phenolischen Hydroxyfunktion, Nitrierung eines Phenylkerns und Reduktion der aromatischen Nitrogruppe zur aromatischen Aminogruppe.

Zur Illustration solcher Herstellungsmethoden für die neuen Verbindungen der Formel III dienen die in dem angeschlossenen Schemata 1 bis 9 aufgeführten Reaktionen. Die Substituenten $R^1$ bis $R^8$ haben die unter Formel I gegebenen Bedeutungen.

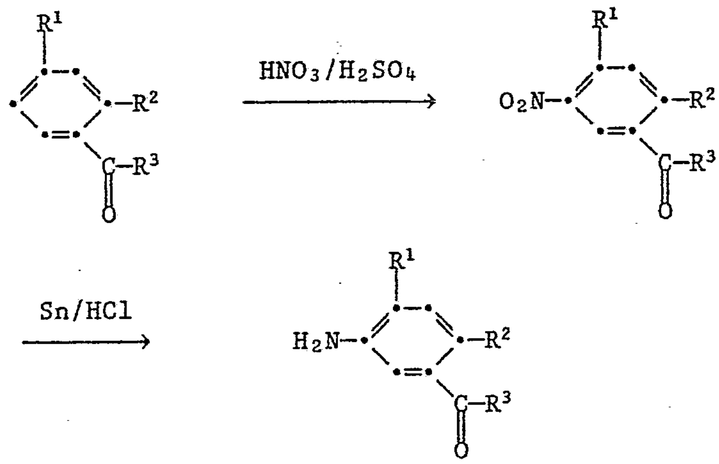


Schema 2:

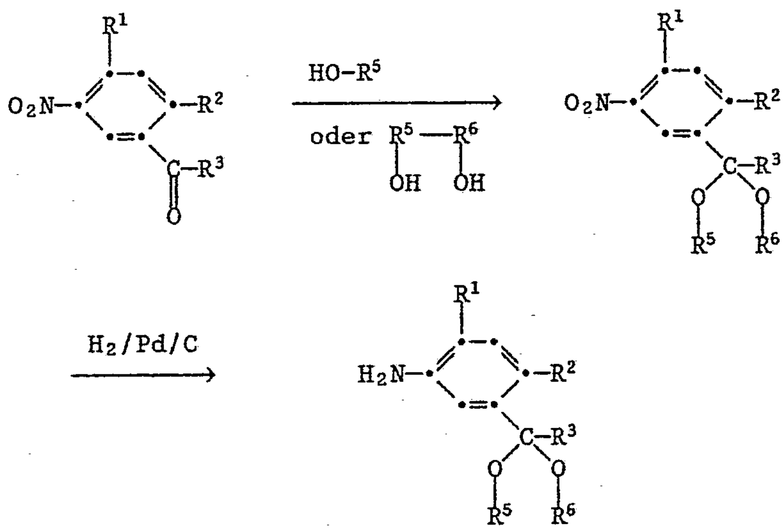

Schema 3:

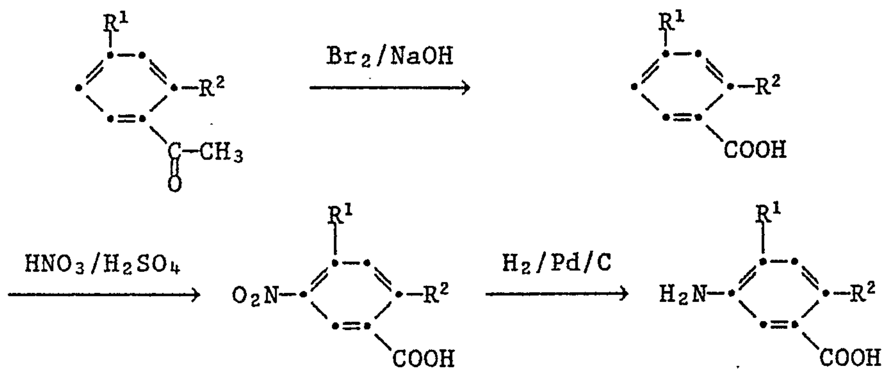


Schema 4:

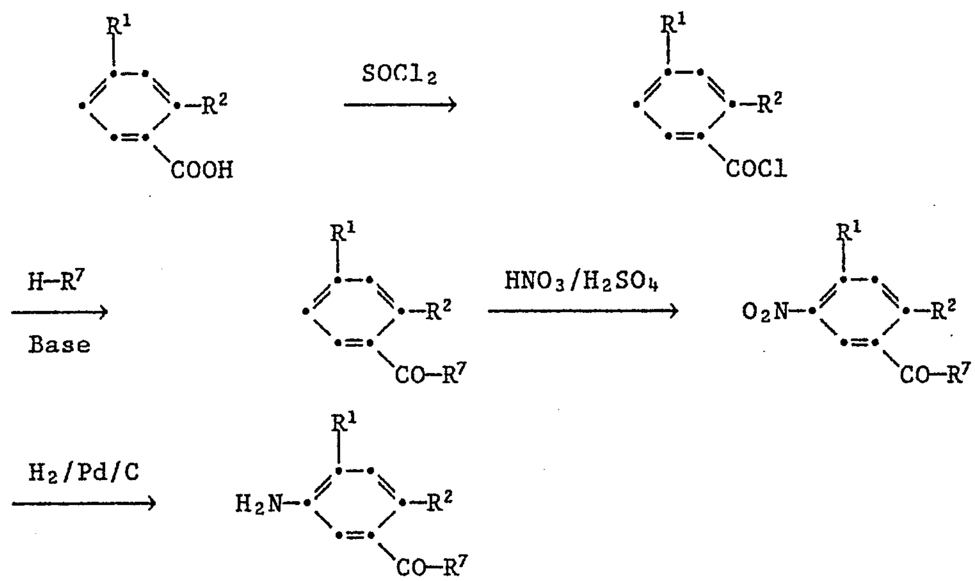

Schema 5: Hal: Chlor, Brom

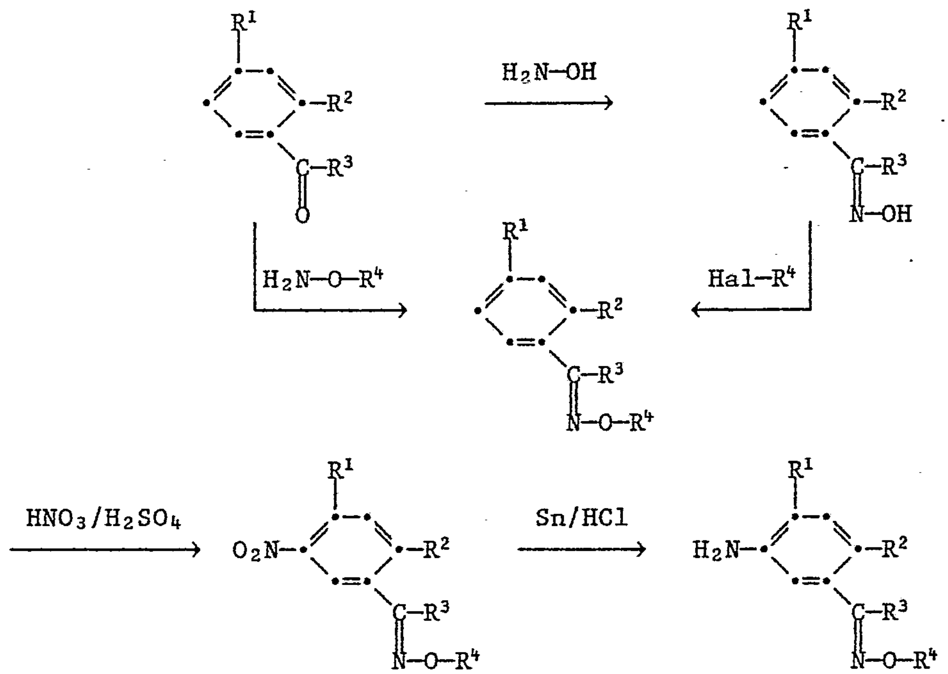


Schema 6: Hal: Chlor, Brom

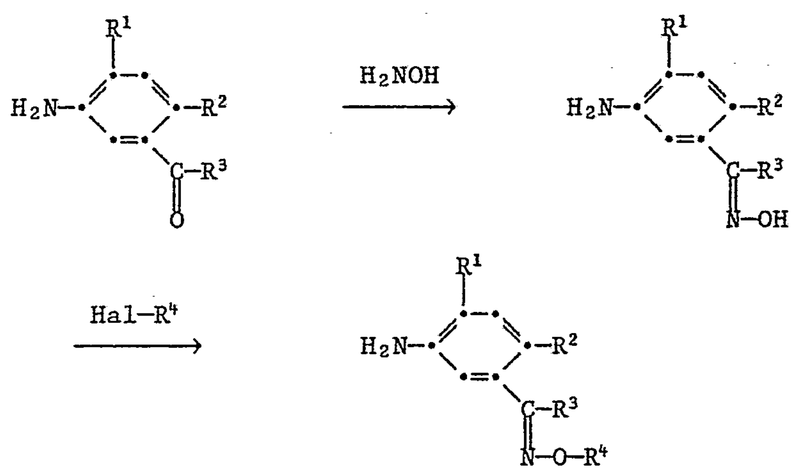

Schema 7: Hal: Chlor, Brom; $M^{\oplus}$: $Na^{\oplus}$, $K^{\oplus}$

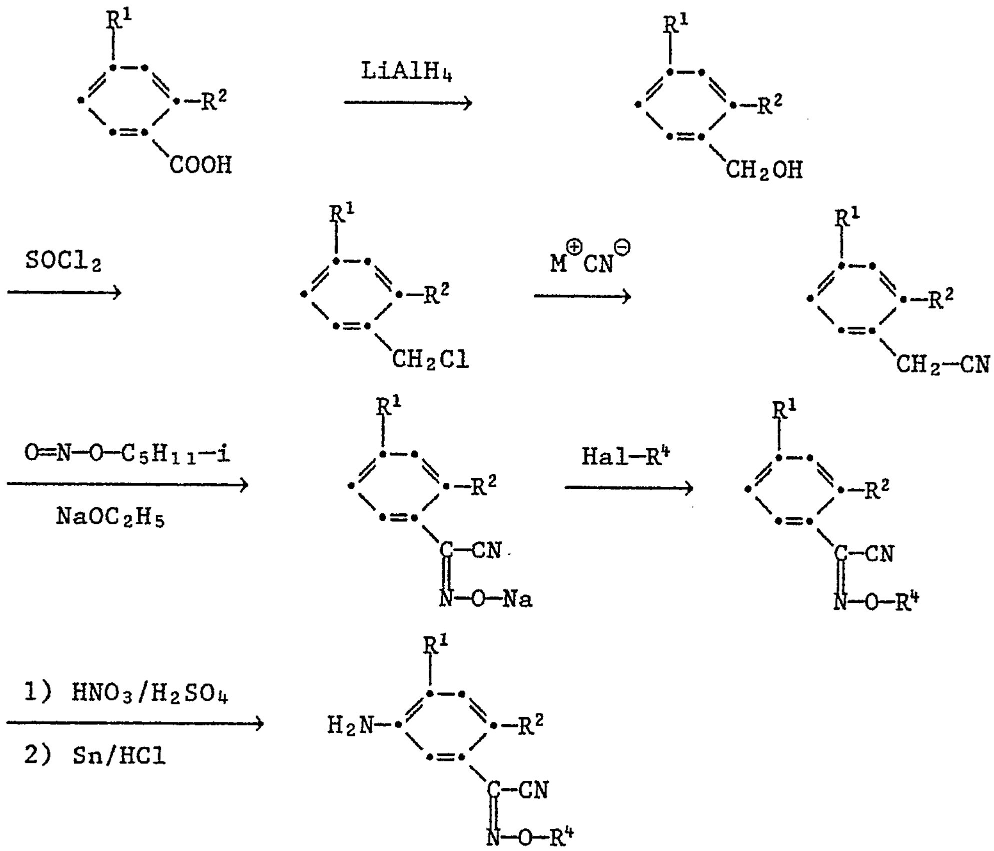


Schema 8: Hal: Chlor, Brom

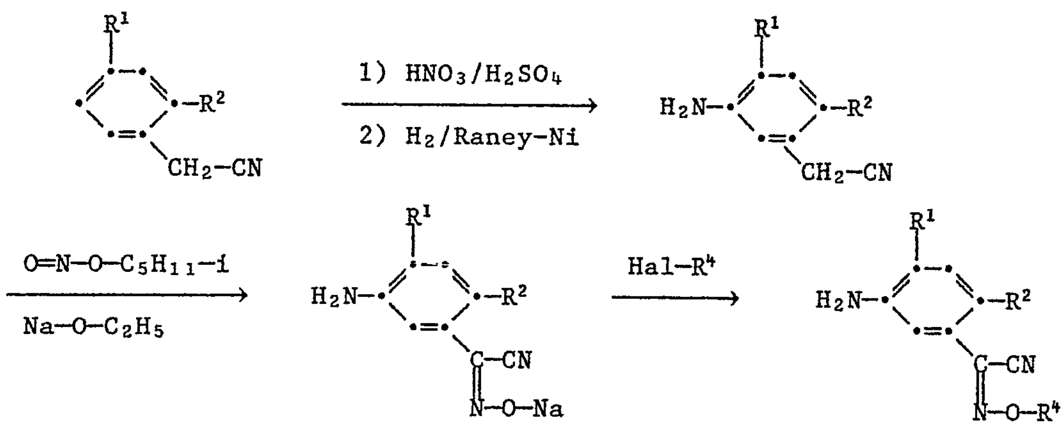

Schema 9: Hal: Chlor, Brom

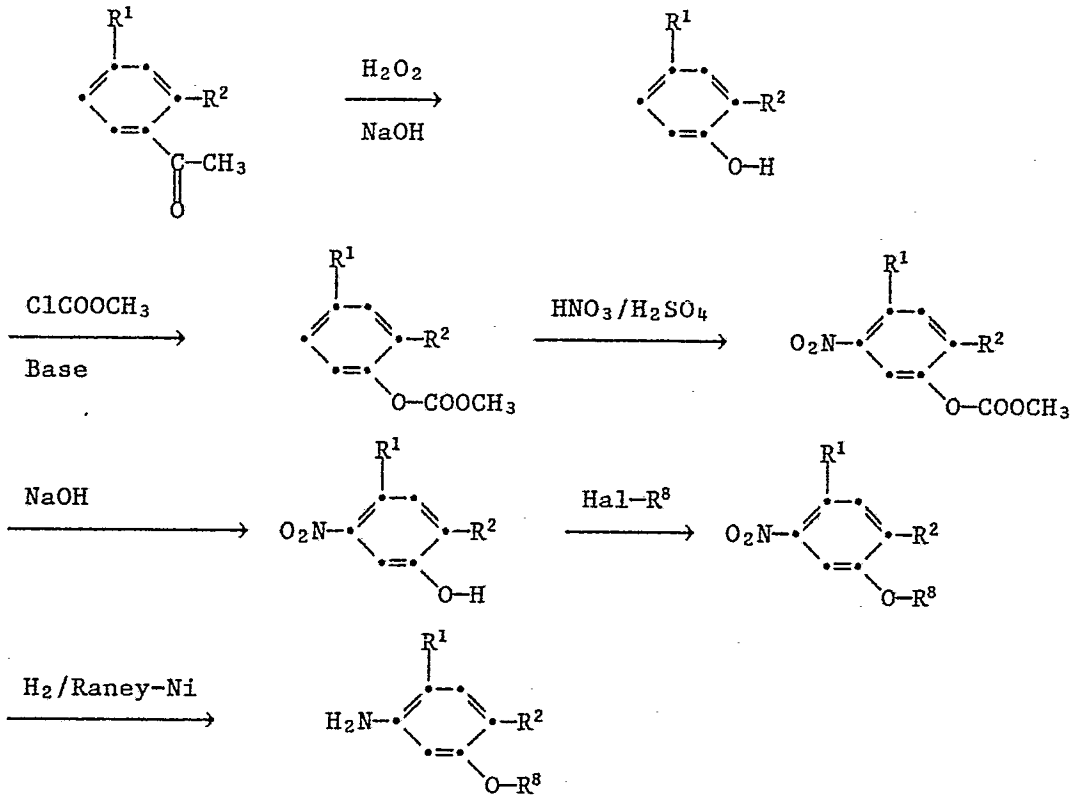


Obwohl die oben skizzierte Synthese der Verbindungen der Formel I aus einem Anhydrid der Formel II und einem Anilin der Formel III in allen Fällen durchführbar ist, kann es aus ökonomischen oder verfahrenstechnischen Gründen sinnvoll sein, bestimmte Verbindungen der Formel I in andere Derivate der Formel I zu überführen. Dabei kommen für den Fachmann bekannte Verfahren, wie beispielsweise Oxidation, Reduktion, Veresterung, Verseifung oder Amidierung in Frage. Einige Beispiele für derartige Umwandlungen von bestimmten Wirkstoffen der Formel I in andere Wirkstoffe der Formel I sind in den folgenden Schemata 10 bis 12 aufgeführt.

Schema 10:

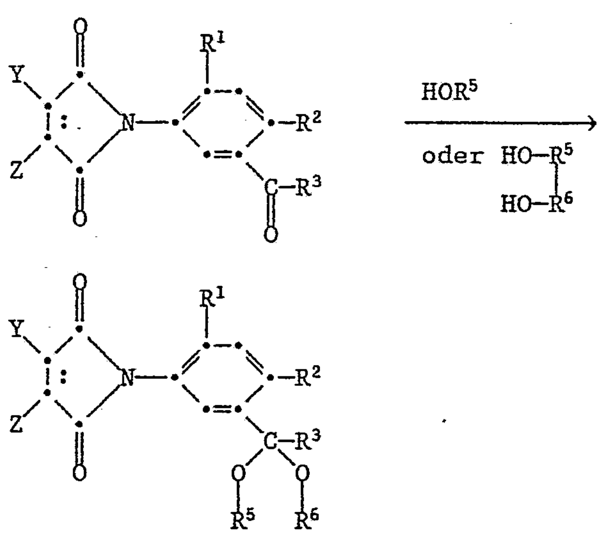

Schema 11:

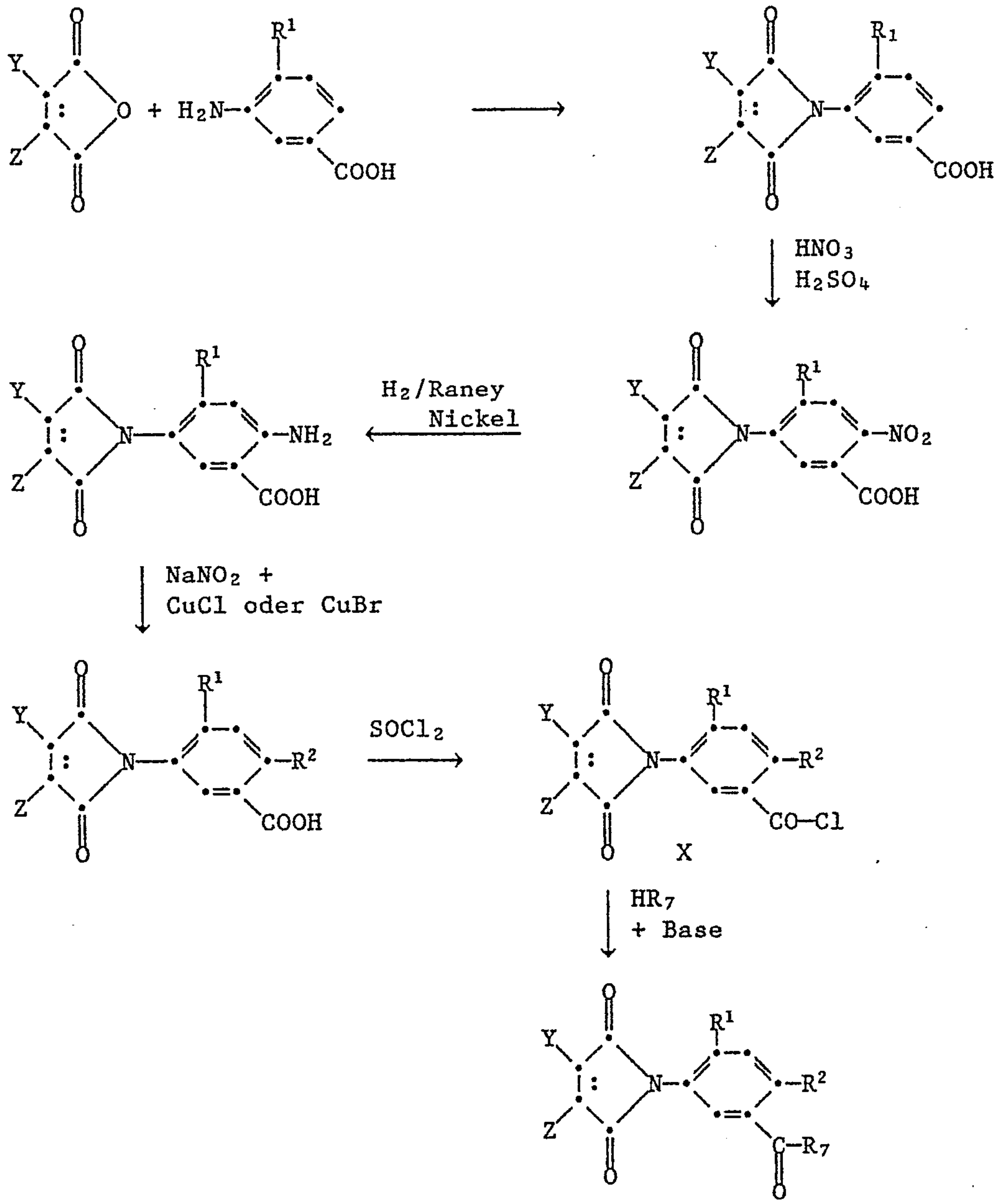


Die Säurechloride der Formel X, in denen $R^1$ Wasserstoff oder Fluor, $R^2$ Chlor oder Brom, Y $C_1$-$C_8$-Alkyl, Z Wasserstoff oder $C_1$-$C_8$-Alkyl bedeuten, sind wichtige Zwischenprodukte bei der Herstellung von Imiden der Formel I. Sie und ihre Herstellung sind ebenfalls ein Gegenstand dieser Erfindung.

Schema 12: Hal: Chlor, Brom

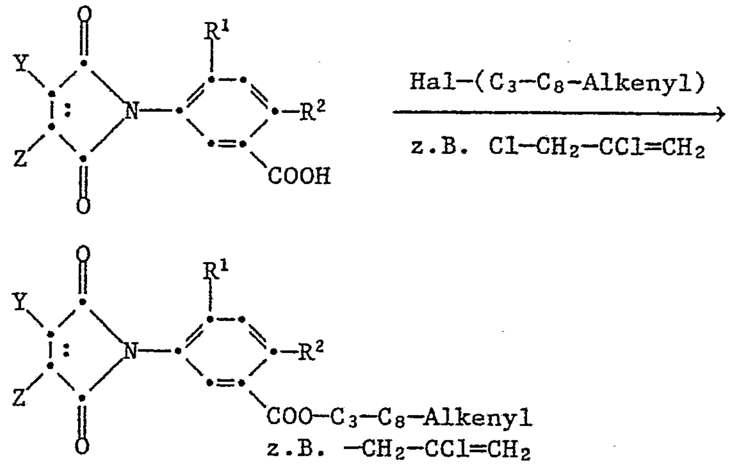


Schema 13:

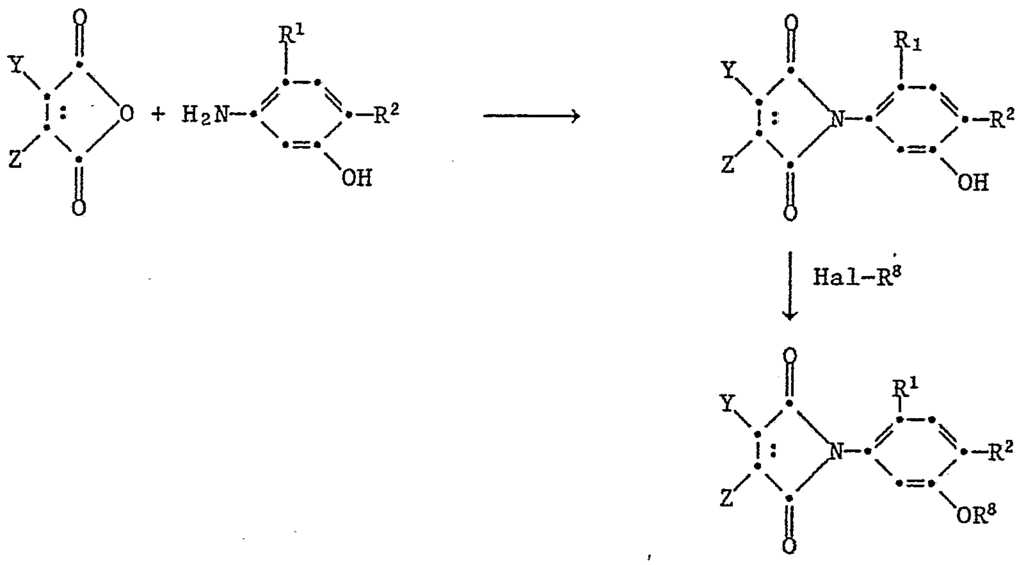


Die erfindungsgemässen Wirkstoffe der Formel I sind stabile Verbindungen, bei deren Handhabung keine besonderen Vorsichtsmassnahmen notwendig sind.

Wenn die Succinimid-Verbindungen der Formel I nicht direkt aus Succinimiden und Phenylverbindungen synthetisiert werden, können sie auch aus den entsprechenden Maleinderivaten gewonnen werden. Die Ueberführung der Maleinsäureimide zu den entsprechenden Succinimiden geschieht am vorteilhaftesten durch Hydrieren mittels Wasserstoff in Gegenwart eines Edelmetallkatalysators wie z.B. Platinoxid. Die Hydrierung kann bei Normaldruck durchgeführt werden.

Je Nach der Stellung der Substituenten Y und Z können die Verbindungen der Formel I in ihrer E- oder Z-Form auftreten, d.h. in optischen (bei Succinimiden) oder geometrischen (bei Maleinimiden) Isomeren vorhanden sein. Die Reste $R^3$, $R^4$, $R^7$ und $R^8$ können asymmetrische Kohlenstoffatome enthalten und solche Reste enthaltende Verbindungen der Formel I ihrerseits in optisch aktive Isomere aufgelöst, respektive als solche synthetisiert werden. Die Stereoisomere der Formel I sind ebenfalls Gegenstand dieser Erfindung.

Die Verbindungen der Formel I sind hochaktive Pflanzenwirkstoffe, welche sich bei geeigneten Aufwandmengen hervorragend als Selektivherbizide zur Unkrautbekämpfung in Nutzpflanzenkulturen eignen. Das heisst, bei diesen Alufwandmengen zeichnen sich die Wirkstoffe der Formel I durch gute selektiv-hebizide Eigenschaft gegen Unkräuter aus. Kulturpflanzen wie Roggen, Gerste, Hafer, Weizen, Mais, Hirse, Reis, Baumwolle und Soja bleiben bei niedrigen Aufwandmengen praktisch ungeschädigt. Bei gesteigerten Aufwandmengen werden die Kulturpflanzen nur geringfügig in ihrem Wachstum beeinflusst. Werden sehr

hohe Aufwandmengen appliziert, entfalten die Substanzen der Formel I totalherbizide Eigenschaften.

Die selektiv-herbizide Wirkung der erfindungsgemässen Verbindungen wird sowhol bei der preemergenten als auch der postemergenten Anwendung festgestellt. Diese Wirkstoffe können daher im Vorauflaufverfahren und im Nachauflaufverfahren zur selektiven Unkrautbekämpfung gleichermassen mit gutem Erfolg verwendet werden.

Die Erfindung betrifft auch herbizide Mittel, welche einen neuen Wirkstoff der Formel I enthalten, sowie Verfahren zur pre- und postemergenten Unkrautbekämpfung.

Die Verbindungen der Formel I werden in unveränderter Form oder vorzugsweise als Mittel zusammen mit den in der Formeulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwaserstoffe wie Cyclohexan oder Paraffine, Alkohole und Glykole sowie deren Aether und Ester, wie Aethanol, Aethylenglykol, Aethylenglykolmonomethyl-oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäuremethyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches.

Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 10 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette, die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)äthylammoniumbromid.

Die in der Formulierungstechnik gebräuchlichen Tenside sind u.a. in folgenden Publikationen beschrieben:
"Mc Cutcheon's Detergents and Emulsifiers Annual" Publishing corp., Ridgewood, New Jersey, 1981;
H. Stache, "Tensid-Taschenbuch", 2. Auflage., C. Hanser Verlag, München, Wien, 1981;
M. and J. Ash. "Encyclopedia of Surfactants", Vol. I-III, Chemical Publishing Co., New York, 1980-1981.

Die herbiziden Zubereitungen enthalten in der Regel 0,1 bis 95 %, insbesondere 0,1 bis 80 % Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent).

Emulgierbare Konzentrate:
Aktiver Wirkstoff: 1 bis 20 %, bevorzugt 5 bis 10 %
oberflächenaktive Mittel: 5 bis 30 %, vorzugsweise 10 bis 20 %
flüssige Trägermittel: 50 bis 94 %, vorzugsweise 70 bis 85 %

Stäube:
Aktiver Wirkstoff: 0,1 bis 10 %, vorzugsweise 0,1 bis 1 %
festes Trägermittel: 99,9 bis 90 %, vorzugsweise 99,9 bis 99 %

Suspensions-Konzentrate:
Aktiver Wirkstoff: 5 bis 75 %, vorzugsweise 10 bis 50 %
Wasser: 94 bis 25 %, vorzugsweise 88 bis 30 %
oberflächenaktives Mittel: 1 bis 40 %, vorzugsweise 2 bis 30 %

Benetzbares Pulver:
Aktiver Wirkstoff: 0,5 bis 90 %, vorzugsweise 1 bis 80 %
oberflächenaktives Mittel: 0,5 bis 20 %, vorzugsweise 1 bis 15 %
festes Trägermittel: 5 bis 95 %, vorzugsweise 15 bis 90 %

Granulate:
Aktiver Wirkstoff: 0,5 bis 30 %, vorzugsweise 3 bis 15 %
festes Trägermittel: 99,5 bis 70 %, vorzugsweise 97 bis 85 %

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,001 bis 4 kg AS/ha, vorzugsweise 0,005 bis 1 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele veranschaulichen die Herstellung der Imide der Formel I. Temperaturangaben beziehen sich auf Celsius-Grade.

Beispiel 1:

Herstellung von N-(2-Fluor-5-carboxyphenyl)-2,3-dimethylenmaleinsäureimid (Zwischenprodukt).

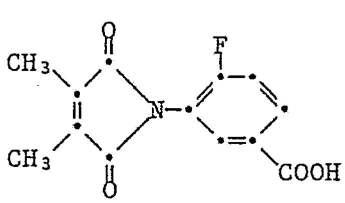

Ein Gemisch von 46,5 g 3-Amino-4-fluorbenzoesäure und 37,8 g Dimethylmaleinsäureanhydrid in 200 ml Eisessig wird während 12 Stunden bei einer Temperatur des Oelbades von 130-40° gerührt. Nach dem Abkühlen wird das Gemisch auf Eiswasser gegossen, der angefallene Niederschlag abgenutscht, mit Wasser gewaschen und getrocknet. Durch Umkritstallisieren aus Aethanol/Wasser erhält man 70,3 g N-(2-Fluor-5-carboxyphenyl)-2,3-dimethylmaleinsäureimid, welches bei 238-240° schmilzt.

Beispiel 2:

Herstellung von N-(2-Fluor-4-nitro-5-carboxylphenyl)-2,3-dimethylmaleinsäureimid (Zwischenprodukt).

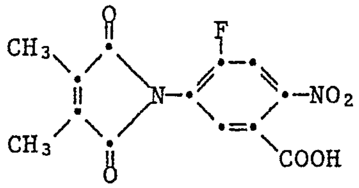

26,3 g N-(2-Fluor-5-carboxyphenyl)-2,3-dimethylmaleinsäureimid werden bei 5° portionsweise in 230 ml 96%ige Schwefelsäure eingetragen. Dann wird zu diesem Gemisch unter starkem Rühren und bei gleichbleibender Temperatur 5 ml 100%ige Salpetersäure zugetropft. Das Ganze wird über Nacht bei Raumtemperatur weitergerührt und dann auf Eis gegossen. Das ausgefallene Produkt wird abgenutscht, mit Wasser gewaschen, getrocknet und aus Acetonitril umkristallisiert. Man erhält so 28 g N-(2-Fluor-5-carboxy-4-nitrophenyl)-2,3-dimethylmaleinsäureimid, welches einen Schmelzpunkt von 264-265° hat.

Beispiel 3:

Herstellung von N-(2-Fluor-4-amino-5-carboxyphenyl)-2,3-dimethylmaleinsäureimid (Zwischenprodukt).

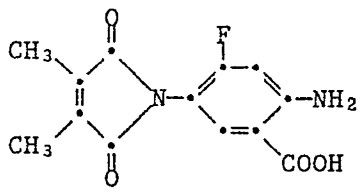

67,8 g N-(2-Fluor-4-nitro-5-carboxyphenyl)-2,3-dimethylmaleinsäureimid werden in 1000 ml Tetrahydrofuran bei einer Temperatur von 20-25° in Gegenwart von 14 g Raney-Nickel-Katalysator unter Normaldruck mit Wasserstoff hydriert. Nachdem die stöchiometrische Menge Wasserstoff verbraucht worden ist, wird der Katalysator abgetrennt und die Lösung eingedampft. Man erhält so 56,7 g N-(2-Fluor-4-amino-5-carboxyphenyl)-2,3-dimethylmaleinsäureimid mit einem Schmelzpunkt von 270°.

Beispiel 4:

Herstellung von N-(2-Fluor-4-chlor-5-carboxyphenyl)-2,3-dimethylmaleinsäureimid.

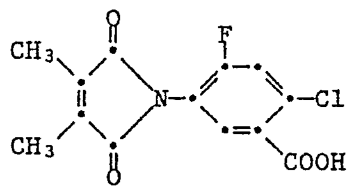

11,4 g N-(2-Fluor-4-amino-5-carboxyphenyl)-2,3-dimethylmaleinsäureimid werden in 60 ml Eisessig und 60 ml 96%iger Schwefelsäure bei einer Temperatur von 25-30° portionsweise eingetragen und das Ganze 1 Stunde lang bei dieser Temperatur gerührt. Anschliessend wird die Lösung auf 5° gerührt und tropfenweise mit einer wässrigen Lösung von 2,9 g Natriumnitrit in 17 ml Wasser versetzt. Die so erhaltene Diazolösung wird unter kräftigem Rühren bei 30° portionsweise zu einer Lösung von 4,5 g Kupfer-I-chlorid in 32 ml konzentrierte Salzsäure gegeben. Nach Beendigung der Stickstoffentwicklung wird das Reaktionsgemisch noch 30 Minuten bei 60° gerührt und dann auf Eis gegossen. Das ausgefallene Produkt wird abgenutscht, mit Wasser gewaschen und aus Methanol/Wasser umkristallisiert. Man erhält 9,8 g N-(2-Fluor-4-chlor-5-carboxyphenyl)-2,3-dimethylmaleinsäureimid mit einem Schmelzpunkt von 230-231°.

Beispiel 5:

Herstellung von N-(2-Fluor-4-chlor-5-chlorocarbonyl-phenyl)-2,3-dimethylmaleinsäureimid (Zwischenprodukt).

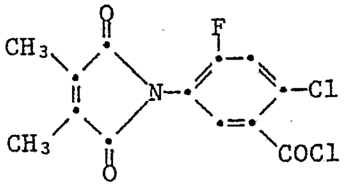

Ein Gemisch von 6 g N-(2-Fluor-4-chlor-5-carboxyphenyl)-2,3-dimethylmaleinimid, 0,5 ml Dimethylformamid, 2 ml Thionylchlorid und 50 ml Toluol werden während 4 Stunden am Rückfluss erhitzt. Nach dem Abkühlen wird am Vakuum eingedampft. Man erhält 6 g N-(2-Fluor-4-chlor-5-chlorcarbonylphenyl)-2,3-dimethylmaleinsäureimid mit einem Schmelzpunkt von 123-124°.

Beispiel 6:

Herstellung von N-(2-Fluor-4-chlor-5-isopropoxycarbonylphenyl)-2,3-dimethylmaleinsäureimid.

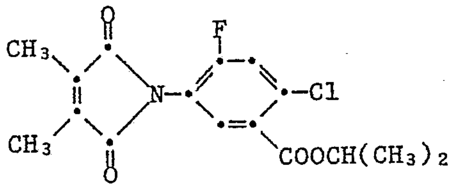

Zu einer Mischung von 4 ml Isopropanol, 4 ml Triäthylamin und 100 ml Toluol tropft man bei Raumtemperatur unter Rühren 6 g N-(2-Fluor-4-chlor-5-chlorcarbonylphenyl)-2,3-dimethylmaleinsäureimid zu. Nach einer Reaktionszeit von 6 Stunden wird das Triäthylaminhydrochlorid durch Filtrieren abgetrennt und das Filtrat eingedampft. Man erhält aus dem Rückstand durch Umkristallisieren aus Aethanol/Wasser 4,2 g N-(2-Fluor-4-chlor-5-isopropoxycarbonylphenyl)-2,3-dimethylmaleinsäureimid mit einem Schmelzpunkt von 161-162°.

Beispiel 7:

Herstellung von N-[2-Fluor-4-chlor-5-(3'-chlorallyloxycarbonyl)-phenyl]-2,3-maleinsäureimid.

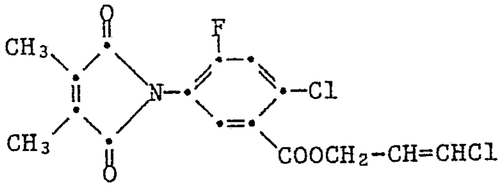

Ein Gemisch von 3 g N-(2-Fluor-4-chlor-5-carboxyphenyl)-2,3-dimethylmaleinsäureimid, 4,5 g Kaliumcarbonat, 1 ml 1,3-Dichlorpropen und 50 ml 2-Butanon wird für 16 Stunden zum Rückfluss erhitzt. Der Niederschlag wird abgetrennt und das Filtrat eingedampft. Man erhält 3,3 g N-[2-Fluor-4-chlor-5-(3'-chlorallyloxycarbonyl)-phenyl]-2,3-dimethylmaleinsäureimid in Form eines Oeles.
Brechnungsindex $\eta_D^{24}$ 1,5640.

Beispiel 8:

Herstellung von N-(2-Fluor-5-methylcarbonylphenyl)-2,3-dimethylmaleinsäureimid (Zwischenprodukt).

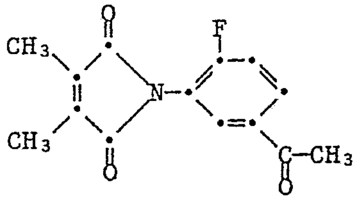

Ein Gemisch von 3 g 3-Amino-4-fluoracetophenon, 2,5 g Dimethylmaleinsäureanhydrid und 50 ml Eisessig wird über Nacht am Rückfluss erhitzt. Das Reaktionsgemisch wird dann auf Eis gegossen der ausgefallene Niederschlag abgenutscht, mit Wasser gewaschen und getrocknet. Man erhält so 2,3 g N-(2-Fluor-5-methylcarbonylphenyl)-2,3-dimethylmaleinsäureimid mit einem Schmelzpunkt von 130-131°. Duch Nitrieren, Reduzieren und Umsetzen mit Natriumnitrit und Kupfer-I-chlorid entsprechend der Beispiele 2, 3 und 4 kann aus dieser Verbindung das N-(2-Fluor-4-chlor-5-methylylcarbonyl)-2,3-dimethylmaleinsäureimid, Smp. 95-97° hergestellt werden.

Beispiel 9:

Herstellung von N-(2-Fluor-4-chlorphenyl)-2,3-dimethylmaleinsäureimid.

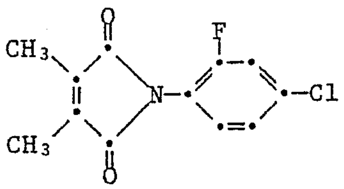

Ein Gemisch von 126 g (1,0 Mol) Dimethylmaleinsäureanhydrid, 145,5 g (1,0 Mol) 2-Fluor-4-chloranilin und 2 g 4-Dimethylaminopyridin wird in 800 ml o-Xylol 16 Stunden mit einem Wasserabscheider am Rückfluss erhitzt. Anschliessend wird im Vakuum zur Trockne verdampft, der Rückstand in einem 1:1 Gemisch von Essigsäure-äthylester:Aether gelöst und je 1 Mal mit 1 N Salzsäure, 1 N Natronlauge und Wasser gewaschen. Nach dem Trocknen über Natriumsulfat und Abdampfen des Lösungsmittels wird der Rückstand (227,5 g) aus Methanol umkristallisiert. Auf diese Weise erhält man 211,1 g (84 % d.Th.) des Titelproduktes als farblose Kristalle, welche bei 96-97° schmelzen.

Beispiel 10:

Herstellung von N-(2-Fluor-4-chlor-5-isopropoxyphenyl)-2,3-dimethyl-maleinsäureimid.

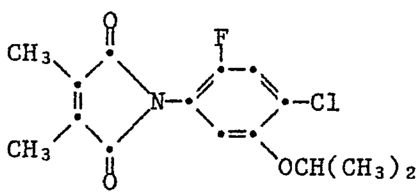

Ein Gemisch von 20,4 g (0.1 Mol) 2-Fluor-4-chlor-5-isopropoxyanilin und 12,6 g (0.1 Mol) Dimethylmaleinsäureanhydrid in 100 ml Eisessig wird während 6 Stunden bei einer Temperatur von 130-140° gerührt. Dann lässt man auf Raumtemperatur abkühlen und engt die fast schwarze Reaktionslösung am Rotationsverdampfer ein. Der Rückstand wird in Essigester aufgenommen und die Lösung nacheinander mit Wasser, 1 M Sodalösung, Wasser und konz. Salzsole gewaschen. Dann wird über Natriumsulfat getrocknet, filtriert und am Vakuum eingeengt. Das zurückbleibende dunkle Oel wird aus Aethanol unter Zuhilfenahme von Aktivkohle Kristallisiert. Man erhält so 17,6 g Titelprodukt (56,5 % der Theorie) welches bei 95-97° schmilzt.

Beispiel 11:

Herstellung von N-(2-Fluor-4-chlorphenyl)-2,3-dimethylsuccinimid.

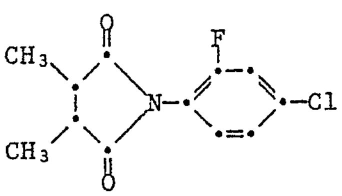

Ein Gemisch von 7,5 g (0,03 Mol) N-(4-Chlor-2-fluorphenyl)-2,3-dimethylmaleinsäureimid und 400 mg Platinoxid ($PtO_2$) wird in 75 ml Essigsäure-äthylester bei Raumtemperatur und Normaldruck mit Wasserstoff hydriert. Nach ca. 5 Stunden ist die Reduktion beendet. Anschliessend wird der Katalysator abfiltriert und das Filtrat im Vakuum eingeengt. Als Rückstand verbleiben 7,6 g (99 % d.Th) cis-N-(2-Fluor-4-chlorphenyl)-2,3-di-

methylsuccinimid als gelbliches, im Dünnschichtchromatogramm einheitliches Oel.
$^1$H-NMR (300 $MH_2$, $CDCl_3$) 1,34 (d, $2xCH_3$); 3,12 (m, CH-CH); 7,2 (m, 3 Ar. H).

Beispiel 12:

Herstellung von N-(2-Fluor-4-chlor-5-isopropoxyphenyl)-2,3-dimethylsuccinimid.

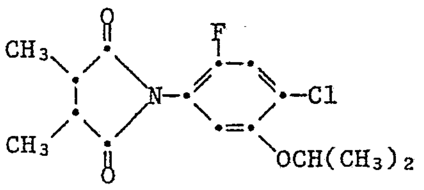

Ein Gemisch von 9,4 g (0,03 Mol) N-(2-Fluor-4-chlor-5-isopropoxyphenyl)-2,3-dimethylmaleinsäureimid und 500 mg Platinoxid ($PtO_2$) wird in 100 ml Essigsäure-äthylester bei Raumtemperatur und Normaldruck mit Wasserstoff hydriert. Nach ca. 5 Stunden, wenn die Wasserstoffaufnahme beendet ist, wird der Katalysator abfiltriert und das Filtrat zur Trockne eingeengt. Es verbleibt ein fast farbloses, im Dünnschichtchromatogramm einheitliches Oel 7,9 g (85 % d.Th) welches aus Isopropyläther kristallisiert. Das so erhaltene cis-N-(2-Fluor-4-chlor-5-isopropoxyphenyl)-2,3-dimethylsuccinimid hat einen Schmelzpunkt von 101-102°.

Beispiel 13:

Herstellung von N-(2-Fluor-4-chlor-5-isopropoxyphenyl)-2-äthyl-3-methylmaleinsäureimid.

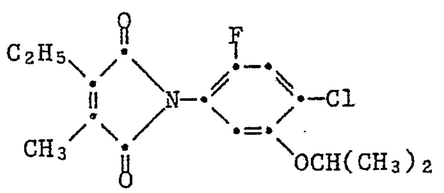

In einer Destillationsapparatur werden 5 g 2-Ethyl-3-methylmaleinsäureanhydrid, 7,3 g 2-Fluor-4-chlor-5-isopropoxyanilin und 0,61 g 4-Dimethylamino-pyridin in 100 ml Xylol zusammengegeben und das Lösungsmittel langsam abdestilliert. Nach 5 Stunden ist die Reaktion beendet. Das noch vorhandene Xylol wird am Wasserstrahlvakuum entfernt. Den dunklen Rückstand nimmt man in Aetylacetat auf, extrahiert diesen mit 2 M Salzsäure, Wasser, 10%iger Natriumbicarbonatlösung und zuletzt mit gesättigter Kochsalzlösung. Nach dem Trocknen über Natriumsulfat wird der Essisäureäthylester am Rotationsverdampfer abdestilliert, und der Rückstand mit Hexan:Essigester 1:1 über eine Kieselgelsäule chromatographiert. Man erhält, nach Verdampfen der Lösungsmittel 5 g der Titelverbindung als rotes Oel mit Berechnungsindex $n_D^{22}$ = 1,5351.

Beispiel 14:

Herstellung von N-(2-Fluor-4-chlor-5-hydroxyphenyl)-2,3-dimethylmaleinsäureimid.

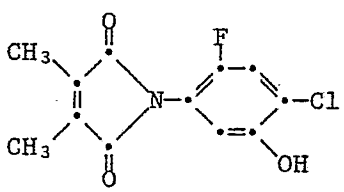

Ein Gemisch von 16,1 g 2-Fluor-4-chlor-5-hydroxy-anilin und 12,6 g Dimethylmaleinsäurehydrid in 100 ml Propionsäure wird während 12 Stunden bei einer Temperatur von 150° gerührt. Dann lässt man abkühlen und engt die Reaktionslösung ein. Der Rückstand wird aus Methanol umkristallisiert. Man erhält 27,3 g N-(2-Fluor-4-chlor-5-hydroxyphenyl)-2,3-dimethylmaleinsäureimid mit einem Schmelzpunkt von 170-171°.

Beispiel 15:

Herstellung von N-(2-Fluor-4-chlor-5-propargyloxyphenyl)-2,3-dimethylmaleinsäureimid.

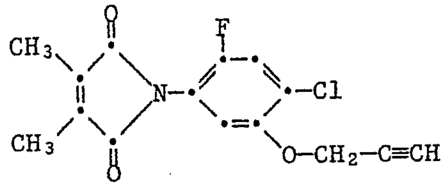

Ein Gemisch von 5,4 g N-(2-Fluor-4-chlor-5-hydroxyphenyl)-2,3-dimethylmaleinsäureimid, 2,4 g Propargylbromid und 4 g Kaliumcarbonat wird in 100 ml Butanon-2 während 2 Stunden bei einer Temperatur von 100° gerührt. Dann lässt man abkühlen, nutscht den Niederschlag ab und engt die Reaktionslösung ein. Nach Kristallisation des Rückstandes erhält man 4,5 g N-(2-Fluor-4-chlor-5-propargylphenyl)-2,3-dimethylmaleinsäureimid mit einem Schmelzpunkt von 149-150°.

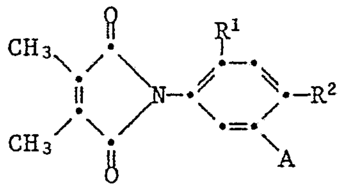

Tabelle 1

| Nr. | $R^1$ | $R^2$ | A |
|---|---|---|---|
| 1.01 | F | Cl | $COCH_3$ |
| 1.02 | F | Cl | $COCH_2Cl$ |
| 1.03 | F | Cl | $COCF_3$ |
| 1.04 | F | | $COCH_3$ |
| 1.05 | F | Cl | $C(CH_3)=N-OH$ |
| 1.06 | F | Cl | $C(CH_3)=N-OCH_3$ |
| 1.07 | F | Cl | $C(CN)=N-OH$ |
| 1.08 | F | Br | $C(CN)=N-OC_2H_5$ |
| 1.09 | F | Cl | $C(CN)=NOCH(CH_3)COOCH_3$ |
| 1.10 | F | Cl | 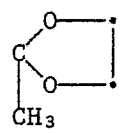 |
| 1.11 | F | Cl | 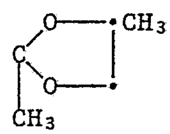 |
| 1.12 | F | Cl | 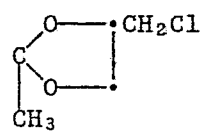 |
| 1.13 | F | Cl | COOH Smp. 230-231° |
| 1.14 | F | Br | COOH Smp. 231-233° |
| 1.15 | F | Cl | $COOCH_3$ Smp. 137-138° |
| 1.16 | F | Cl | $COOCH(CH_3)_2$ Smp. 101-102° |
| 1.17 | H | Cl | COOH |
| 1.18 | H | Cl | $COOCH_3$ |
| 1.19 | H | Cl | $COOCH(CH_3)_2$ |
| 1.20 | F | Cl | $COSCH_2COOCH_3$ Smp. 78-81° |
| 1.21 | F | Cl | $COSCH_2COOCH_2(CH_3)_2$ |
| 1.22 | F | Cl | $COSCH(CH_3)COOCH_3$ $n_D^{23}$= 1,5668 |

Tabelle 1 (Fortsetzung)

| Nr. | $R^1$ | $R^2$ | A | |
|---|---|---|---|---|
| 1.23 | F | Cl | $COSCH(CH_3)COOCH_2CH{=}CH_2$ | |
| 1.24 | F | Cl | OH | Smp. 170-171° |
| 1.25 | F | Br | OH | |
| 1.26 | F | Cl | $OCH_3$ | |
| 1.27 | F | Cl | $OCH(CH_3)_2$ | Smp. 95-97° |
| 1.28 | F | Br | $OCH_2CH{=}CH_2$ | |
| 1.29 | F | Cl | $OCH_2CH{=}CHCl$ | |
| 1.30 | F | Cl | $OCH_2CCl{=}CH_2$ | |
| 1.31 | F | Cl | $OCH_2COOCH_3$ | |
| 1.32 | F | Cl | $OCH_2COOCH(CH_3)_2$ | |
| 1.33 | F | Cl | SH | |
| 1.34 | F | Br | SH | |
| 1.35 | F | Cl | $SCH_3$ | |
| 1.36 | F | Cl | $SCH(CH_3)_2$ | |
| 1.37 | F | Cl | $SCH_2CH{=}CHCl$ | |
| 1.38 | F | Cl | $SCH_2COOH$ | Smp. 165-167° |
| 1.39 | F | Br | $SCH_2COOH$ | |
| 1.40 | F | Cl | $SCH(CH_3)COOH$ | |
| 1.41 | F | Cl | $SCH_2COOCH_3$ | |
| 1.42 | F | Cl | $SCH(CH_3)COOCH_3$ | |
| 1.43 | F | Cl | $OCCl{=}CHCl$ | Smp. 127-130° |
| 1.44 | F | Cl | $OCF_3$ | Smp. 79-82° |
| 1.45 | F | Cl | $OCClF_2$ | Smp. 90-93° |
| 1.46 | F | H | COOH | Smp. 238-240° |
| 1.47 | H | Br | $COOCH(CH_3)_2$ | Smp. 93-95° |
| 1.48 | F | Br | $COOOCH(CH_3)_2$ | Smp. 104-105° |
| 1.49 | F | Br | $COSCH(CH_3)COOCH_3$ | Smp. 82-83° |
| 1.50 | F | Cl | $COSCH(CH_3)COOC_2H_5$ | $n_D^{23}$= 1,5529 |
| 1.51 | F | Cl | $COOCH_2CH(CH_3)_2$ | Smp. 83-84° |
| 1.52 | F | Cl | $COOC_2H_5$ | Smp. 101-102° |
| 1.53 | F | Cl | $COOCH(CH_3)COOCH_3$ | $n_D^{23}$= 1,5243 |
| 1.54 | F | Br | $COOCH(CH_3)COOCH_3$ | $n_D^{23}$= 1,5398 |

Tabelle 1 (Fortsetzung)

| Nr. | $R^1$ | $R^2$ | A | |
|---|---|---|---|---|
| 1.55 | F | Br | $COOCH(CH_3)CH_2SC_2H_5$ | $n_D^{23} = 1,5301$ |
| 1.56 | F | Br | $COOCH(CH_3)CH_2SCH_3$ | $n_D^{23} = 1,5670$ |
| 1.57 | F | Br | $COOCH(CH_3)CH_2SCH(CH_3)_2$ | $n_D^{23} = 1,5271$ |
| 1.58 | F | Br | $COSCH(CH_3)COOC_2H_5$ | $n_D^{23} = 1,5530$ |
| 1.59 | F | Br | $COSCH(CH_3)COOCH(CH_3)_2$ | $n_D^{23} = 1,5252$ |
| 1.60 | F | Cl | $COOCH(CH_3)CH_2SC_2H_5$ | $n_D^{23} = 1,5489$ |
| 1.61 | F | Cl | $COOCH_2CH=CHCl$ | $n_D^{24} = 1,5640$ |
| 1.62 | F | Cl | $COOCH(CH_3)COOC_2H_5$ | $n_D^{24} = 1,5319$ |
| 1.63 | F | Cl | $COOCH(CH_3)CH_2SC(CH_3)_3$ | $n_D^{23} = 1,5373$ |
| 1.64 | F | Cl | $COSCH(CH_3)COOCH(CH_3)_2$ | $n_D^{23} = 1,5389$ |
| 1.65 | F | Cl | $COOCH_2CH=CH_2$ | $n_D^{25} = 1,5532$ |
| 1.66 | F | Cl | $COSCH_2COOC_2H_5$ | $n_D^{23} = 1,5639$ |
| 1.67 | F | Cl | $COOCH(CH_3)CH_2SC_3H_7-n$ | $n_D^{25} = 1,5425$ |
| 1.68 | F | Cl | $COOCH(CH_3)CH_2SCH(CH_3)_2$ | $n_D^{24} = 1,5432$ |
| 1.69 | F | Cl | $COOCH(C_3\ )CH_2SC_4H_9-n$ | $n_D^{25} = 1,5309$ |
| 1.70 | F | H | $COCH_3$ | Smp. 130-131° |
| 1.71 | F | Cl | $COOCH(CH_3)CH_2SCH_3$ | $n_D^{25} = 1,5406$ |
| 1.72 | F | Cl | $OCH_2C\equiv CH$ | Smp. 149-150° |
| 1.73 | F | Cl | $OCH(CH_3)COOCH_3$ | Smp. 117-118° |
| 1.74 | F | Cl | $-COOCH_2-\underset{Cl}{C}=CH_2$ | $n_D^{25} = 1,5422$ |
| 1.75 | F | Cl | $-COOCH_2-\underset{Br}{C}=CH_2$ | |
| 1.76 | F | Cl | $-COO-CH_2-CH=CCl-CH_3$ | |
| 1.77 | F | Cl | $-COO-CH_2-\underset{Br}{C}=CHBr$ | |
| 1.78 | F | Cl | $-COOCH_2-C\equiv CH$ | |
| 1.79 | F | Br | $-COOCH_2-CH=CCl\cdot CH_3$ | |
| 1.80 | F | Br | $-COOCH_2-CCl=CH_2$ | |
| 1.81 | F | Br | $-COOCH_2-CH=CHCl$ | |
| 1.82 | F | Br | $-COOCH_2-CBr=CH_2$ | |
| 1.83 | F | Br | $-O-CH_2-C\equiv CH$ | |
| 1.84 | F | Cl | $-O-CH(CH_3)-CH_2-S-CH_3$ | $n_D^{23} = 1,5468$ |

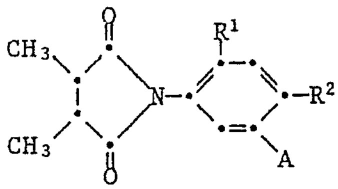

Tabelle 2

| Nr. | $R^1$ | $R^2$ | A |
|---|---|---|---|
| 2.01 | F | Cl | $COCH_3$ |
| 2.02 | F | Cl | $COCH_2Cl$ |
| 2.03 | F | Cl | $COCF_3$ |
| 2.04 | F | | $COCH_3$ |
| 2.05 | F | Cl | $C(CH_3)=N-OH$ |
| 2.06 | F | Cl | $C(CH_3)=N-OCH_3$ |
| 2.07 | F | Cl | $C(CN)=N-OH$ |
| 2.08 | F | Br | $C(CN)=N-OC_2H_5$ |
| 2.09 | F | Cl | $C(CN)=NOCH(CH_3)COOCH_3$ |
| 2.10 | F | Cl | 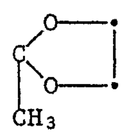 |
| 2.11 | F | Cl | 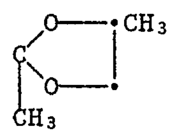 |
| 2.12 | F | Cl | 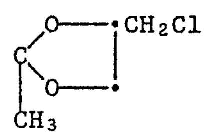 |
| 2.13 | F | Cl | COOH |
| 2.14 | F | Br | COOH |
| 2.15 | F | Cl | $COOCH_3$ |
| 2.16 | F | Cl | $COOCH(CH_3)_2$ |
| 2.17 | H | Cl | COOH |
| 2.18 | H | Cl | $COOCH_3$ |
| 2.19 | H | Cl | $COOCH(CH_3)_2$ |
| 2.20 | F | Cl | $COSCH_2COOCH_3$ |
| 2.21 | F | Cl | $COSCH_2COOCH_2(CH_3)_2$ |
| 2.22 | F | Cl | $COSCH_2(CH_3)COOCH_3$ |

Tabelle 2 (Fortsetzung)

| Nr. | $R^1$ | $R^2$ | A | |
|---|---|---|---|---|
| 2.23 | F | Cl | $COSCH(CH_3)COOCH_2CH=CH_2$ | |
| 2.24 | F | Cl | OH | |
| 2.25 | F | Br | OH | |
| 2.26 | F | Cl | $OCH_3$ | |
| 2.27 | F | Cl | $OCH(CH_3)_2$ | Smp. 102° |
| 2.28 | F | Br | $OCH_2CH=CH_2$ | |
| 2.29 | F | Cl | $OCH_2CH=CHCl$ | |
| 2.30 | F | Cl | $OCH_2CCl=CH_2$ | |
| 2.31 | F | Cl | $OCH_2COOCH_3$ | |
| 2.32 | F | Cl | $OCH_2COOCH(CH_3)_2$ | |
| 2.33 | F | Cl | SH | |
| 2.34 | F | Br | SH | |
| 2.35 | F | Cl | $SCH_3$ | |
| 2.36 | F | Cl | $SCH(CH_3)_2$ | |
| 2.37 | F | Cl | $SCH_2CH=CHCl$ | |
| 2.38 | F | Cl | $SCH_2COOH$ | |
| 2.39 | F | Br | $SCH_2COOH$ | |
| 2.40 | F | Cl | $SCH(CH_3)COOH$ | |
| 2.41 | F | Cl | $SCH_2COOCH_3$ | |
| 2.42 | F | Cl | $SCH(CH_3)COOCH_3$ | |
| 2.43 | F | Cl | H | Oel |
| 2.44 | F | Cl | $OCH_2C\equiv CH$ | |
| 2.45 | F | Cl | $OCH(CH_3)COOCH_3$ | |

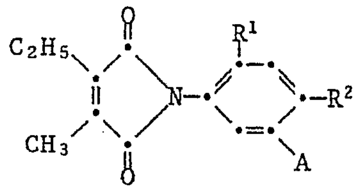

Tabelle 3

| Nr. | $R^1$ | $R^2$ | A | |
|---|---|---|---|---|
| 3.01 | F | Cl | $COCH_3$ | |
| 3.02 | F | Cl | $COCH_2Cl$ | |
| 3.03 | F | Cl | $COCF_3$ | |
| 3.04 | F | | $COCH_3$ | |
| 3.05 | F | Cl | $C(CH_3)=N-OH$ | |
| 3.06 | F | Cl | $C(CH_3)=N-OCH_3$ | |
| 3.07 | F | Cl | $C(CN)=N-OH$ | |
| 3.08 | F | Br | $C(CN)=N-OC_2H_5$ | |
| 3.09 | F | Cl | $C(CN)=NOCH(CH_3)COOCH_3$ | |
| 3.10 | F | Cl | 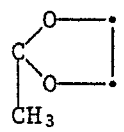 | |
| 3.11 | F | Cl | 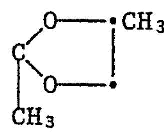 | |
| 3.12 | F | Cl | 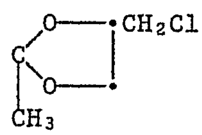 | |
| 3.13 | F | Cl | COOH | Smp. 165° |
| 3.14 | F | Br | COOH | |
| 3.15 | F | Cl | $COOCH_3$ | |
| 3.16 | F | Cl | $COOCH(CH_3)_2$ | Smp. 68-69° |
| 3.17 | H | Cl | COOH | |
| 3.18 | H | Cl | $COOCH_3$ | |
| 3.19 | H | Cl | $COOCH(CH_3)_2$ | $n_D^{22} = 1.5458$ |
| 3.20 | F | Cl | $COSCH_2COOCH_3$ | |
| 3.21 | F | Cl | $COSCH_2COOCH_2(CH_3)_2$ | |
| 3.22 | F | Cl | $COSCH_2(CH_3)COOCH_3$ | |

<u>Tabelle 3</u> (Fortsetzung)

| Nr. | $R^1$ | $R^2$ | A |
|---|---|---|---|
| 3.23 | F | Cl | $COSCH(CH_3)COOCH_2CH=CH_2$ |
| 3.24 | F | Cl | OH Smp. 74-75° |
| 3.25 | F | Br | OH |
| 3.26 | F | Cl | $OCH_3$ |
| 3.27 | F | Cl | $OCH(CH_3)_2$ $n_D^{22} = 1,5351$ |
| 3.28 | F | Br | $OCH_2CH=CH_2$ |
| 3.29 | F | Cl | $OCH_2CH=CHCl$ $n_D^{23} = 1,5572$ |
| 3.30 | F | Cl | $OCH_2CCl=CH_2$ |
| 3.31 | F | Cl | $OCH_2COOCH_3$ |
| 3.32 | F | Cl | $OCH_2COOCH(CH_3)_2$ |
| 3.33 | F | Cl | SH |
| 3.34 | F | Br | SH |
| 3.35 | F | Cl | $SCH_3$ |
| 3.36 | F | Cl | $SCH(CH_3)_2$ |
| 3.37 | F | Cl | $SCH_2CH=CHCl$ |
| 3.38 | F | Cl | $SCH_2COOH$ |
| 3.39 | F | Br | $SCH_2COOH$ |
| 3.40 | F | Cl | $SCH(CH_3)COOH$ |
| 3.41 | F | Cl | $SCH_2COOCH_3$ |
| 3.42 | F | Cl | $SCH(CH_3)COOCH_3$ |
| 3.43 | F | Cl | $OCH_2C{\equiv}CH$ Smp. 98-99° |
| 3.44 | F | Cl | $OCH(CH_3)COOCH_3$ $n_D^{24}=1,5308$ |
| 3.45 | F | Cl | $COOCH(CH_3)-CH_2-S-CH_3$ |
| 3.46 | F | Cl | $COOCH(CH_3)-CH_2-S-C_2H_5$ |
| 3.47 | F | Cl | $COO-CH_2-CCl=CH_2$ |
| 3.48 | F | Cl | $COOCH_2-CH=CHCl$ |
| 3.49 | F | Cl | $COOCH_2-CH=CCl-CH_3$ |
| 3.50 | F | Cl | $COOCH_2-CBr=CH_2$ |
| 3.60 | F | Cl | $COOCH_2-CBr=CHBr$ |
| 3.61 | F | Br | $COOCH_2-CCl=CH_2$ |
| 3.62 | F | Br | $COOCH_2-CH=CHCl$ |
| 3.63 | F | Br | $COOCH_2-CH=CCl-CH_3$ |
| 3.64 | F | Br | $COOCH_2-CBr=CH_2$ |
| 3.65 | F | Br | $-O-CH_2-C{\equiv}CH$ |
| 3.66 | F | Br | $-O-CH(CH_3)-COOCH_3$ |

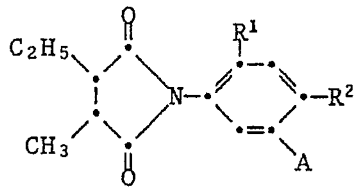

Tabelle 4

| Nr. | $R^1$ | $R^2$ | A |
|---|---|---|---|
| 4.01 | F | Cl | $COCH_3$ |
| 4.02 | F | Cl | $COCH_2Cl$ |
| 4.03 | F | Cl | $COCF_3$ |
| 4.04 | F | | $COCH_3$ |
| 4.05 | F | Cl | $C(CH_3)=N-OH$ |
| 4.06 | F | Cl | $C(CH_3)=N-OCH_3$ |
| 4.07 | F | Cl | $C(CN)=N-OH$ |
| 4.08 | F | Br | $C(CN)=N-OC_2H_5$ |
| 4.09 | F | Cl | $C(CN)=NOCH(CH_3)COOCH_3$ |
| 4.10 | F | Cl | 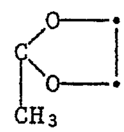 |
| 4.11 | F | Cl | 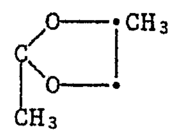 |
| 4.12 | F | Cl | 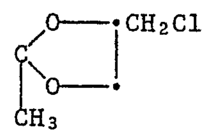 |
| 4.13 | F | Cl | COOH |
| 4.14 | F | Br | COOH |
| 4.15 | F | Cl | $COOCH_3$ |
| 4.16 | F | Cl | $COOCH(CH_3)_2$ |
| 4.17 | H | Cl | COOH |
| 4.18 | H | Cl | $COOCH_3$ |
| 4.19 | H | Cl | $COOCH(CH_3)_2$ |
| 4.20 | F | Cl | $COSCH_2COOCH_3$ |
| 4.21 | F | Cl | $COSCH_2COOCH_2(CH_3)_2$ |
| 4.22 | F | Cl | $COSCH_2(CH_3)COOCH_3$ |

Tabelle 4 (Fortsetzung)

| Nr. | $R^1$ | $R^2$ | A |
|---|---|---|---|
| 4.23 | F | Cl | $COSCH(CH_3)COOCH_2CH=CH_2$ |
| 4.24 | F | Cl | OH |
| 4.25 | F | Br | OH |
| 4.26 | F | Cl | $OCH_3$ |
| 4.27 | F | Cl | $OCH(CH_3)_2$ |
| 4.28 | F | Br | $OCH_2CH=CH_2$ |
| 4.29 | F | Cl | $OCH_2CH=CHCl$ |
| 4.30 | F | Cl | $OCH_2CCl=CH_2$ |
| 4.31 | F | Cl | $OCH_2COOCH_3$ |
| 4.32 | F | Cl | $OCH_2COOCH(CH_3)_2$ |
| 4.33 | F | Cl | SH |
| 4.34 | F | Br | SH |
| 4.35 | F | Cl | $SCH_3$ |
| 4.36 | F | Cl | $SCH(CH_3)_2$ |
| 4.37 | F | Cl | $SCH_2CH=CHCl$ |
| 4.38 | F | Cl | $SCH_2COOH$ |
| 4.39 | F | Br | $SCH_2COOH$ |
| 4.40 | F | Cl | $SCH(CH_3)COOH$ |
| 4.41 | F | Cl | $SCH_2COOCH_3$ |
| 4.42 | F | Cl | $SCH(CH_3)COOCH_3$ |
| 4.43 | F | Cl | $OCH_2C\equiv CH$ |
| 4.44 | F | Cl | $OCH(CH_3)COOCH_3$ |

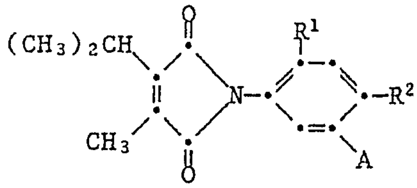

Tabelle 5

| Nr. | $R^1$ | $R^2$ | A |
|---|---|---|---|
| 5.01 | F | Cl | $COCH_3$ |
| 5.02 | F | Cl | $COCH_2Cl$ |
| 5.03 | F | Cl | $COCF_3$ |
| 5.04 | F | | $COCH_3$ |
| 5.05 | F | Cl | $C(CH_3)=N-OH$ |
| 5.06 | F | Cl | $C(CH_3)=N-OCH_3$ |
| 5.07 | F | Cl | $C(CN)=N-OH$ |
| 5.08 | F | Br | $C(CN)=N-OC_2H_5$ |
| 5.09 | F | Cl | $C(CN)=NOCH(CH_3)COOCH_3$ |
| 5.10 | F | Cl | 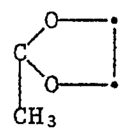 |
| 5.11 | F | Cl | 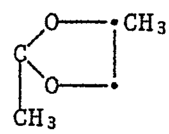 |
| 5.12 | F | Cl | 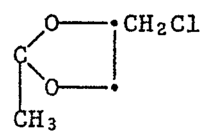 |
| 5.13 | F | Cl | COOH |
| 5.14 | F | Br | COOH |
| 5.15 | F | Cl | $COOCH_3$ |
| 5 16 | F | Cl | $COOCH(CH_3)_2$ |
| 5.17 | H | Cl | COOH |
| 5.18 | H | Cl | $COOCH_3$ |
| 5.19 | H | Cl | $COOCH(CH_3)_2$ |
| 5.20 | F | Cl | $COSCH_2COOCH_3$ |
| 5.21 | F | Cl | $COSCH_2COOCH_2(CH_3)_2$ |
| 5.22 | F | Cl | $COSCH_2(CH_3)COOCH_3$ |

Tabelle 5 (Fortsetzung)

| Nr. | $R^1$ | $R^2$ | A | |
|---|---|---|---|---|
| 5.23 | F | Cl | $COSCH(CH_3)COOCH_2CH=CH_2$ | |
| 5.24 | F | Cl | OH | |
| 5.25 | F | Br | OH | |
| 5.26 | F | Cl | $OCH_3$ | |
| 5.27 | F | Cl | $OCH(CH_3)_2$ | $n_D^{23} = 1,5298$ |
| 5.28 | F | Br | $OCH_2CH=CH_2$ | |
| 5.29 | F | Cl | $OCH_2CH=CHCl$ | |
| 5.30 | F | Cl | $OCH_2CCl=CH_2$ | |
| 5.31 | F | Cl | $OCH_2COOCH_3$ | |
| 5.32 | F | Cl | $OCH_2COOCH(CH_3)_2$ | |
| 5.33 | F | Cl | SH | |
| 5.34 | F | Br | SH | |
| 5.35 | F | Cl | $SCH_3$ | |
| 5.36 | F | Cl | $SCH(CH_3)_2$ | |
| 5.37 | F | Cl | $SCH_2CH=CHCl$ | |
| 5.38 | F | Cl | $SCH_2COOH$ | |
| 5.39 | F | Br | $SCH_2COOH$ | |
| 5.40 | F | Cl | $SCH(CH_3)COOH$ | |
| 5.41 | F | Cl | $SCH_2COOCH_3$ | |
| 5.42 | F | Cl | $SCH(CH_3)COOCH_3$ | |
| 5.43 | F | Cl | $OCH_2C\equiv CH$ | |
| 5.44 | F | Cl | $OCH(CH_3)COOCH_3$ | |

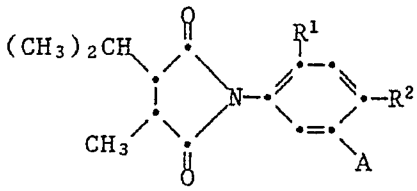

Tabelle 6

| Nr. | $R^1$ | $R^2$ | A |
|---|---|---|---|
| 6.01 | F | Cl | $COCH_3$ |
| 6.02 | F | Cl | $COCH_2Cl$ |
| 6.03 | F | Cl | $COCF_3$ |
| 6.04 | F | | $COCH_3$ |
| 6.05 | F | Cl | $C(CH_3)=N-OH$ |
| 6.06 | F | Cl | $C(CH_3)=N-OCH_3$ |
| 6.07 | F | Cl | $C(CN)=N-OH$ |
| 6.08 | F | Br | $C(CN)=N-OC_2H_5$ |
| 6.09 | F | Cl | $C(CN)=NOCH(CH_3)COOCH_3$ |
| 6.10 | F | Cl | 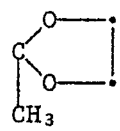 |
| 6.11 | F | Cl | 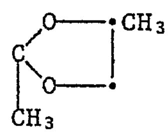 |
| 6.12 | F | Cl | 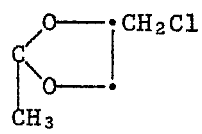 |
| 6.13 | F | Cl | COOH |
| 6.14 | F | Br | COOH |
| 6.15 | F | Cl | $COOCH_3$ |
| 6.16 | F | Cl | $COOCH(CH_3)_2$ |
| 6.17 | H | Cl | COOH |
| 6.18 | H | Cl | $COOCH_3$ |
| 6.19 | H | Cl | $COOCH(CH_3)_2$ |
| 6.20 | F | Cl | $COSCH_2COOCH_3$ |
| 6.21 | F | Cl | $COSCH_2COOCH_2(CH_3)_2$ |
| 6.22 | F | Cl | $COSCH_2(CH_3)COOCH_3$ |

Tabelle 6 (Fortsetzung)

| Nr. | $R^1$ | $R^2$ | A | |
|---|---|---|---|---|
| 6.23 | F | Cl | $COSCH(CH_3)COOCH_2CH=CH_2$ | |
| 6.24 | F | Cl | OH | |
| 6.25 | F | Br | OH | |
| 6.26 | F | Cl | $OCH_3$ | |
| 6.27 | F | Cl | $OCH(CH_3)_2$ | Smp. 71-73° |
| 6.28 | F | Br | $OCH_2CH=CH_2$ | |
| 6.29 | F | Cl | $OCH_2CH=CHCl$ | |
| 6.30 | F | Cl | $OCH_2CCl=CH_2$ | |
| 6.31 | F | Cl | $OCH_2COOCH_3$ | |
| 6.32 | F | Cl | $OCH_2COOCH(CH_3)_2$ | |
| 6.33 | F | Cl | SH | |
| 6.34 | F | Br | SH | |
| 6.35 | F | Cl | $SCH_3$ | |
| 6.36 | F | Cl | $SCH(CH_3)_2$ | |
| 6.37 | F | Cl | $SCH_2CH=CHCl$ | |
| 6.38 | F | Cl | $SCH_2COOH$ | |
| 6.39 | F | Br | $SCH_2COOH$ | |
| 6.40 | F | Cl | $SCH(CH_3)COOH$ | |
| 6.41 | F | Cl | $SCH_2COOCH_3$ | |
| 6.42 | F | Cl | $SCH(CH_3)COOCH_3$ | |
| 6.43 | F | Cl | $OCH_2C{\equiv}CH$ | |
| 6.44 | F | Cl | $OCH(CH_3)COOCH_3$ | |

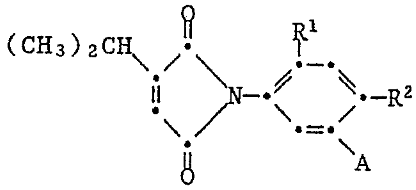

Tabelle 7

| Nr. | $R^1$ | $R^2$ | A |
|---|---|---|---|
| 7.01 | F | Cl | $COCH_3$ |
| 7.02 | F | Cl | $COCH_2Cl$ |
| 7.03 | F | Cl | $COCF_3$ |
| 7.04 | F | | $COCH_3$ |
| 7.05 | F | Cl | $C(CH_3)=N-OH$ |
| 7.06 | F | Cl | $C(CH_3)=N-OCH_3$ |
| 7.07 | F | Cl | $C(CN)=N-OH$ |
| 7.08 | F | Br | $C(CN)=N-OC_2H_5$ |
| 7.09 | F | Cl | $C(CN)=NOCH(CH_3)COOCH_3$ |
| 7.10 | F | Cl | 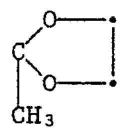 |
| 7.11 | F | Cl | 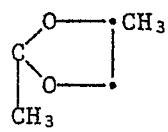 |
| 7.12 | F | Cl | 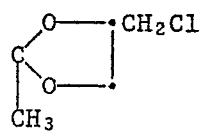 |
| 7.13 | F | Cl | COOH |
| 7.14 | F | Br | COOH |
| 7.15 | F | Cl | $COOCH_3$ |
| 7.16 | F | Cl | $COOCH(CH_3)_2$ |
| 7.17 | H | Cl | COOH |
| 7.18 | H | Cl | $COOCH_3$ |
| 7.19 | H | Cl | $COOCH(CH_3)_2$ |
| 7.20 | F | Cl | $COSCH_2COOCH_3$ |
| 7.21 | F | Cl | $COSCH_2COOCH_2(CH_3)_2$ |
| 7.22 | F | Cl | $COSCH_2(CH_3)COOCH_3$ |

Tabelle 7 (Fortsetzung)

| Nr. | $R^1$ | $R^2$ | A |
|---|---|---|---|
| 7.23 | F | Cl | $COSCH(CH_3)COOCH_2CH=CH_2$ |
| 7.24 | F | Cl | OH |
| 7.25 | F | Br | OH |
| 7.26 | F | Cl | $OCH_3$ |
| 7.27 | F | Cl | $OCH(CH_3)_2$ |
| 7.28 | F | Br | $OCH_2CH=CH_2$ |
| 7.29 | F | Cl | $OCH_2CH=CHCl$ |
| 7.30 | F | Cl | $OCH_2CCl=CH_2$ |
| 7.31 | F | Cl | $OCH_2COOCH_3$ |
| 7.32 | F | Cl | $OCH_2COOCH(CH_3)_2$ |
| 7.33 | F | Cl | SH |
| 7.34 | F | Br | SH |
| 7.35 | F | Cl | $SCH_3$ |
| 7.36 | F | Cl | $SCH(CH_3)_2$ |
| 7.37 | F | Cl | $SCH_2CH=CHCl$ |
| 7.38 | F | Cl | $SCH_2COOH$ |
| 7.39 | F | Br | $SCH_2COOH$ |
| 7.40 | F | Cl | $SCH(CH_3)COOH$ |
| 7.41 | F | Cl | $SCH_2COOCH_3$ |
| 7.42 | F | Cl | $SCH(CH_3)COOCH_3$ |
| 7.43 | F | Cl | $OCH_2C{\equiv}CH$ |
| 7.44 | F | Cl | $OCH(CH_3)COOCH_3$ |

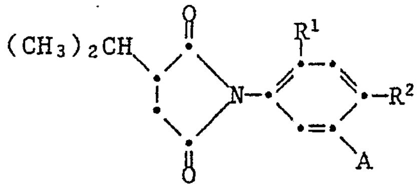

Tabelle 8

| Nr. | $R^1$ | $R^2$ | A |
|---|---|---|---|
| 8.01 | F | Cl | $COCH_3$ |
| 8.02 | F | Cl | $COCH_2Cl$ |
| 8.03 | F | Cl | $COCF_3$ |
| 8.04 | F | | $COCH_3$ |
| 8.05 | F | Cl | $C(CH_3)=N-OH$ |
| 8.06 | F | Cl | $C(CH_3)=N-OCH_3$ |
| 8.07 | F | Cl | $C(CN)=N-OH$ |
| 8.08 | F | Br | $C(CN)=N-OC_2H_5$ |
| 8.09 | F | Cl | $C(CN)=NOCH(CH_3)COOCH_3$ |
| 8.10 | F | Cl | 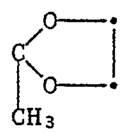 |
| 8.11 | F | Cl | 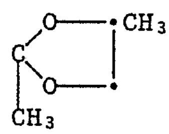 |
| 8.12 | F | Cl | 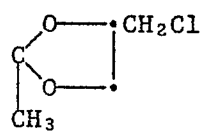 |
| 8.13 | F | Cl | COOH |
| 8.14 | F | Br | COOH |
| 8.15 | F | Cl | $COOCH_3$ |
| 8 16 | F | Cl | $COOCH(CH_3)_2$ |
| 8.17 | H | Cl | COOH |
| 8.18 | H | Cl | $COOCH_3$ |
| 8.19 | H | Cl | $COOCH(CH_3)_2$ |
| 8.20 | F | Cl | $COSCH_2COOCH_3$ |
| 8.21 | F | Cl | $COSCH_2COOCH_2(CH_3)_2$ |
| 8.22 | F | Cl | $COSCH_2(CH_3)COOCH_3$ |

Tabelle 8 (Fortsetzung)

| Nr. | $R^1$ | $R^2$ | A |
|---|---|---|---|
| 8.23 | F | Cl | $COSCH(CH_3)COOCH_2CH{=}CH_2$ |
| 8.24 | F | Cl | OH |
| 8.25 | F | Br | OH |
| 8.26 | F | Cl | $OCH_3$ |
| 8.27 | F | Cl | $OCH(CH_3)_2$ Smp. 119-120° |
| 8.28 | F | Br | $OCH_2CH{=}CH_2$ |
| 8.29 | F | Cl | $OCH_2CH{=}CHCl$ |
| 8.30 | F | Cl | $OCH_2CCl{=}CH_2$ |
| 8.31 | F | Cl | $OCH_2COOCH_3$ |
| 8.32 | F | Cl | $OCH_2COOCH(CH_3)_2$ |
| 8.33 | F | Cl | SH |
| 8.34 | F | Br | SH |
| 8.35 | F | Cl | $SCH_3$ |
| 8.36 | F | Cl | $SCH(CH_3)_2$ |
| 8.37 | F | Cl | $SCH_2CH{=}CHCl$ |
| 8.38 | F | Cl | $SCH_2COOH$ |
| 8.39 | F | Br | $SCH_2COOH$ |
| 8.40 | F | Cl | $SCH(CH_3)COOH$ |
| 8.41 | F | Cl | $SCH_2COOCH_3$ |
| 8.42 | F | Cl | $SCH(CH_3)COOCH_3$ |
| 8.43 | F | Cl | $OCH_2C{\equiv}CH$ |
| 8.44 | F | Cl | $OCH(CH_3)COOCH_3$ |

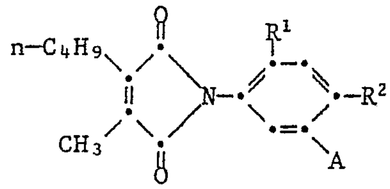

Tabelle 9

| Nr. | $R^1$ | $R^2$ | A |
|---|---|---|---|
| 9.01 | F | Cl | $COCH_3$ |
| 9.02 | F | Cl | $COCH_2Cl$ |
| 9.03 | F | Cl | $COCF_3$ |
| 9.04 | F | | $COCH_3$ |
| 9.05 | F | Cl | $C(CH_3)=N-OH$ |
| 9.06 | F | Cl | $C(CH_3)=N-OCH_3$ |
| 9.07 | F | Cl | $C(CN)=N-OH$ |
| 9.08 | F | Br | $C(CN)=N-OC_2H_5$ |
| 9.09 | F | Cl | $C(CN)=NOCH(CH_3)COOCH_3$ |
| 9.10 | F | Cl | 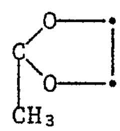 |
| 9.11 | F | Cl | 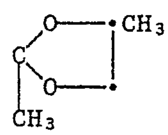 |
| 9.12 | F | Cl | 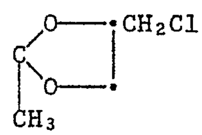 |
| 9.13 | F | Cl | COOH |
| 9.14 | F | Br | COOH |
| 9.15 | F | Cl | $COOCH_3$ |
| 9.16 | F | Cl | $COOCH(CH_3)_2$ |
| 9.17 | H | Cl | COOH |
| 9.18 | H | Cl | $COOCH_3$ |
| 9.19 | H | Cl | $COOCH(CH_3)_2$ |
| 9.20 | F | Cl | $COSCH_2COOCH_3$ |
| 9.21 | F | Cl | $COSCH_2COOCH_2(CH_3)_2$ |
| 9.22 | F | Cl | $COSCH_2(CH_3)COOCH_3$ |

Tabelle 9 (Fortsetzung)

| Nr. | $R^1$ | $R^2$ | A | |
|---|---|---|---|---|
| 9.23 | F | Cl | $COSCH(CH_3)COOCH_2CH=CH_2$ | |
| 9.24 | F | Cl | OH | |
| 9.25 | F | Br | OH | |
| 9.26 | F | Cl | $OCH_3$ | |
| 9.27 | F | Cl | $OCH(CH_3)_2$ | Oel |
| 9.28 | F | Br | $OCH_2CH=CH_2$ | |
| 9.29 | F | Cl | $OCH_2CH=CHCl$ | |
| 9.30 | F | Cl | $OCH_2CCl=CH_2$ | |
| 9.31 | F | Cl | $OCH_2COOCH_3$ | |
| 9.32 | F | Cl | $OCH_2COOCH(CH_3)_2$ | |
| 9.33 | F | Cl | SH | |
| 9.34 | F | Br | SH | |
| 9.35 | F | Cl | $SCH_3$ | |
| 9.36 | F | Cl | $SCH(CH_3)_2$ | |
| 9.37 | F | Cl | $SCH_2CH=CHCl$ | |
| 9.38 | F | Cl | $SCH_2COOH$ | |
| 9.39 | F | Br | $SCH_2COOH$ | |
| 9.40 | F | Cl | $SCH(CH_3)COOH$ | |
| 9.41 | F | Cl | $SCH_2COOCH_3$ | |
| 9.42 | F | Cl | $SCH(CH_3)COOCH_3$ | |
| 9.43 | F | Cl | $OCH_2C{\equiv}CH$ | |
| 9.44 | F | Cl | $OCH(CH_3)COOCH_3$ | |

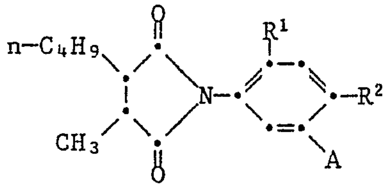

Tabelle 10

| Nr. | $R^1$ | $R^2$ | A |
|---|---|---|---|
| 10.01 | F | Cl | $COCH_3$ |
| 10.02 | F | Cl | $COCH_2Cl$ |
| 10.03 | F | Cl | $COCF_3$ |
| 10.04 | F | | $COCH_3$ |
| 10.05 | F | Cl | $C(CH_3)=N-OH$ |
| 10.06 | F | Cl | $C(CH_3)=N-OCH_3$ |
| 10.07 | F | Cl | $C(CN)=N-OH$ |
| 10.08 | F | Br | $C(CN)=N-OC_2H_5$ |
| 10.09 | F | Cl | $C(CN)=NOCH(CH_3)COOCH_3$ |
| 10.10 | F | Cl | 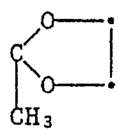 |
| 10.11 | F | Cl | 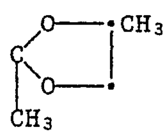 |
| 10.12 | F | Cl | 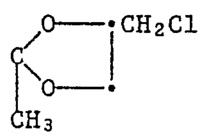 |
| 10.13 | F | Cl | COOH |
| 10.14 | F | Br | COOH |
| 10.15 | F | Cl | $COOCH_3$ |
| 10.16 | F | Cl | $COOCH(CH_3)_2$ |
| 10.17 | H | Cl | COOH |
| 10.18 | H | Cl | $COOCH_3$ |
| 10.19 | H | Cl | $COOCH(CH_3)_2$ |
| 10.20 | F | Cl | $COSCH_2COOCH_3$ |
| 10.21 | F | Cl | $COSCH_2COOCH_2(CH_3)_2$ |
| 10.22 | F | Cl | $COSCH_2(CH_3)COOCH_3$ |

Tabelle 10 (Fortsetzung)

| Nr. | $R^1$ | $R^2$ | A |
|---|---|---|---|
| 10.23 | F | Cl | $COSCH(CH_3)COOCH_2CH{=}CH_2$ |
| 10.24 | F | Cl | OH |
| 10.25 | F | Br | OH |
| 10.26 | F | Cl | $OCH_3$ |
| 10.27 | F | Cl | $OCH(CH_3)_2$ |
| 10.28 | F | Br | $OCH_2CH{=}CH_2$ |
| 10.29 | F | Cl | $OCH_2CH{=}CHCl$ |
| 10.30 | F | Cl | $OCH_2CCl{=}CH_2$ |
| 10.31 | F | Cl | $OCH_2COOCH_3$ |
| 10.32 | F | Cl | $OCH_2COOCH(CH_3)_2$ |
| 10.33 | F | Cl | SH |
| 10.34 | F | Br | SH |
| 10.35 | F | Cl | $SCH_3$ |
| 10.36 | F | Cl | $SCH(CH_3)_2$ |
| 10.37 | F | Cl | $SCH_2CH{=}CHCl$ |
| 10.38 | F | Cl | $SCH_2COOH$ |
| 10.39 | F | Br | $SCH_2COOH$ |
| 10.40 | F | Cl | $SCH(CH_3)COOH$ |
| 10.41 | F | Cl | $SCH_2COOCH_3$ |
| 10.42 | F | Cl | $SCH(CH_3)COOCH_3$ |
| 10.43 | F | Cl | $OCH_2C{\equiv}CH$ |
| 10.44 | F | Cl | $OCH(CH_3)COOCH_3$ |

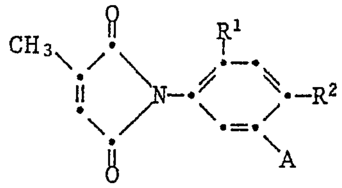

Tabelle 11

| Nr. | $R^1$ | $R^2$ | A |
|---|---|---|---|
| 11.01 | F | Cl | $COCH_3$ |
| 11.02 | F | Cl | $COCH_2Cl$ |
| 11.03 | F | Cl | $COCF_3$ |
| 11.04 | F | | $COCH_3$ |
| 11.05 | F | Cl | $C(CH_3)=N-OH$ |
| 11.06 | F | Cl | $C(CH_3)=N-OCH_3$ |
| 11.07 | F | Cl | $C(CN)=N-OH$ |
| 11.08 | F | Br | $C(CN)=N-OC_2H_5$ |
| 11.09 | F | Cl | $C(CN)=NOCH(CH_3)COOCH_3$ |
| 11.10 | F | Cl | 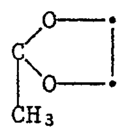 |
| 11.11 | F | Cl | 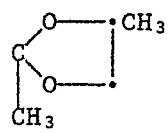 |
| 11.12 | F | Cl | 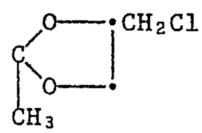 |
| 11.13 | F | Cl | COOH |
| 11.14 | F | Br | COOH |
| 11.15 | F | Cl | $COOCH_3$ |
| 11.16 | F | Cl | $COOCH(CH_3)_2$ |
| 11.17 | H | Cl | COOH |
| 11.18 | H | Cl | $COOCH_3$ |
| 11.19 | H | Cl | $COOCH(CH_3)_2$ |
| 11.20 | F | Cl | $COSCH_2COOCH_3$ |
| 11.21 | F | Cl | $COSCH_2COOCH_2(CH_3)_2$ |
| 11.22 | F | Cl | $COSCH_2(CH_3)COOCH_3$ |

Tabelle 11 (Fortsetzung)

| Nr. | $R^1$ | $R^2$ | A |
|---|---|---|---|
| 11.23 | F | Cl | $COSCH(CH_3)COOCH_2CH=CH_2$ |
| 11.24 | F | Cl | OH |
| 11.25 | F | Br | OH |
| 11.26 | F | Cl | $OCH_3$ |
| 11.27 | F | Cl | $OCH(CH_3)_2$ Smp. 68-70° |
| 11.28 | F | Br | $OCH_2CH=CH_2$ |
| 11.29 | F | Cl | $OCH_2CH=CHCl$ |
| 11.30 | F | Cl | $OCH_2CCl=CH_2$ |
| 11.31 | F | Cl | $OCH_2COOCH_3$ |
| 11.32 | F | Cl | $OCH_2COOCH(CH_3)_2$ |
| 11.33 | F | Cl | SH |
| 11.34 | F | Br | SH |
| 11.35 | F | Cl | $SCH_3$ |
| 11.36 | F | Cl | $SCH(CH_3)_2$ |
| 11.37 | F | Cl | $SCH_2CH=CHCl$ |
| 11.38 | F | Cl | $SCH_2COOH$ |
| 11.39 | F | Br | $SCH_2COOH$ |
| 11.40 | F | Cl | $SCH(CH_3)COOH$ |
| 11.41 | F | Cl | $SCH_2COOCH_3$ |
| 11.42 | F | Cl | $SCH(CH_3)COOCH_3$ |
| 11.43 | F | Cl | $OCH_2C{\equiv}CH$ |
| 11.44 | F | Cl | $OCH(CH_3)COOCH_3$ |

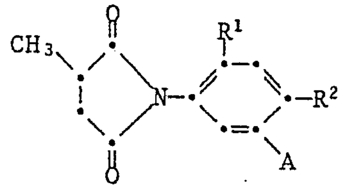

Tabelle 12

| Nr. | $R^1$ | $R^2$ | A |
|---|---|---|---|
| 12.01 | F | Cl | $COCH_3$ |
| 12.02 | F | Cl | $COCH_2Cl$ |
| 12.03 | F | Cl | $COCF_3$ |
| 12.04 | F | | $COCH_3$ |
| 12.05 | F | Cl | $C(CH_3)=N-OH$ |
| 12.06 | F | Cl | $C(CH_3)=N-OCH_3$ |
| 12.07 | F | Cl | $C(CN)=N-OH$ |
| 12.08 | F | Br | $C(CN)=N-OC_2H_5$ |
| 12.09 | F | Cl | $C(CN)=NOCH(CH_3)COOCH_3$ |
| 12.10 | F | Cl | 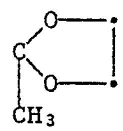 |
| 12.11 | F | Cl | 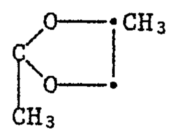 |
| 12.12 | F | Cl | 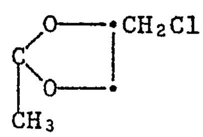 |
| 12.13 | F | Cl | COOH |
| 12.14 | F | Br | COOH |
| 12.15 | F | Cl | $COOCH_3$ |
| 12.16 | F | Cl | $COOCH(CH_3)_2$ |
| 12.17 | H | Cl | COOH |
| 12.18 | H | Cl | $COOCH_3$ |
| 12.19 | H | Cl | $COOCH(CH_3)_2$ |
| 12.20 | F | Cl | $COSCH_2COOCH_3$ |
| 12.21 | F | Cl | $COSCH_2COOCH_2(CH_3)_2$ |
| 12.22 | F | Cl | $COSCH_2(CH_3)COOCH_3$ |

Tabelle 12 (Fortsetzung)

| Nr. | $R^1$ | $R^2$ | A |
|---|---|---|---|
| 12.23 | F | Cl | $COSCH(CH_3)COOCH_2CH=CH_2$ |
| 12.24 | F | Cl | OH |
| 12.25 | F | Br | OH |
| 12.26 | F | Cl | $OCH_3$ |
| 12.27 | F | Cl | $OCH(CH_3)_2$ |
| 12.28 | F | Br | $OCH_2CH=CH_2$ |
| 12.29 | F | Cl | $OCH_2CH=CHCl$ |
| 12.30 | F | Cl | $OCH_2CCl=CH_2$ |
| 12.31 | F | Cl | $OCH_2COOCH_3$ |
| 12.32 | F | Cl | $OCH_2COOCH(CH_3)_2$ |
| 12.33 | F | Cl | SH |
| 12.34 | F | Br | SH |
| 12.35 | F | Cl | $SCH_3$ |
| 12.36 | F | Cl | $SCH(CH_3)_2$ |
| 12.37 | F | Cl | $SCH_2CH=CHCl$ |
| 12.38 | F | Cl | $SCH_2COOH$ |
| 12.39 | F | Br | $SCH_2COOH$ |
| 12.40 | F | Cl | $SCH(CH_3)COOH$ |
| 12.41 | F | Cl | $SCH_2COOCH_3$ |
| 12.42 | F | Cl | $SCH(CH_3)COOCH_3$ |
| 12.43 | F | Cl | $OCH_2C\equiv CH$ |
| 12.44 | F | Cl | $OCH(CH_3)COOCH_3$ |

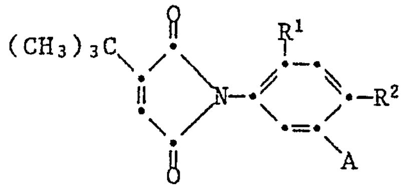

Tabelle 13

| Nr. | $R^1$ | $R^2$ | A |
|---|---|---|---|
| 13.01 | F | Cl | $COCH_3$ |
| 13.02 | F | Cl | $COCH_2Cl$ |
| 13.03 | F | Cl | $COCF_3$ |
| 13.04 | F | | $COCH_3$ |
| 13.05 | F | Cl | $C(CH_3)=N-OH$ |
| 13.06 | F | Cl | $C(CH_3)=N-OCH_3$ |
| 13.07 | F | Cl | $C(CN)=N-OH$ |
| 13.08 | F | Br | $C(CN)=N-OC_2H_5$ |
| 13.09 | F | Cl | $C(CN)=NOCH(CH_3)COOCH_3$ |
| 13.10 | F | Cl | 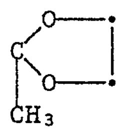 |
| 13.11 | F | Cl | 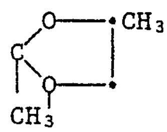 |
| 13.12 | F | Cl | 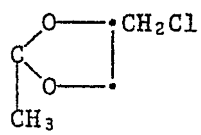 |
| 13.13 | F | Cl | COOH |
| 13.14 | F | Br | COOH |
| 13.15 | F | Cl | $COOCH_3$ |
| 13.16 | F | Cl | $COOCH(CH_3)_2$ |
| 13.17 | H | Cl | COOH |
| 13.18 | H | Cl | $COOCH_3$ |
| 13.19 | H | Cl | $COOCH(CH_3)_2$ |
| 13.20 | F | Cl | $COSCH_2COOCH_3$ |
| 13.21 | F | Cl | $COSCH_2COOCH_2(CH_3)_2$ |
| 13.22 | F | Cl | $COSCH_2(CH_3)COOCH_3$ |

<u>Tabelle 13</u> (Fortsetzung)

| Nr. | $R^1$ | $R^2$ | A | |
|---|---|---|---|---|
| 13.23 | F | Cl | $COSCH(CH_3)COOCH_2CH{=}CH_2$ | |
| 13.24 | F | Cl | OH | |
| 13.25 | F | Br | OH | |
| 13.26 | F | Cl | $OCH_3$ | |
| 13.27 | F | Cl | $OCH(CH_3)_2$ | |
| 13.28 | F | Br | $OCH_2CH{=}CH_2$ | |
| 13.29 | F | Cl | $OCH_2CH{=}CHCl$ | |
| 13.30 | F | Cl | $OCH_2CCl{=}CH_2$ | |
| 13.31 | F | Cl | $OCH_2COOCH_3$ | |
| 13.32 | F | Cl | $OCH_2COOCH(CH_3)_2$ | |
| 13.33 | F | Cl | SH | |
| 13.34 | F | Br | SH | |
| 13.35 | F | Cl | $SCH_3$ | |
| 13.36 | F | Cl | $SCH(CH_3)_2$ | |
| 13.37 | F | Cl | $SCH_2CH{=}CHCl$ | |
| 13.38 | F | Cl | $SCH_2COOH$ | |
| 13.39 | F | Br | $SCH_2COOH$ | |
| 13.40 | F | Cl | $SCH(CH_3)COOH$ | |
| 13.41 | F | Cl | $SCH_2COOCH_3$ | |
| 13.42 | F | Cl | $SCH(CH_3)COOCH_3$ | |
| 13.43 | H | Cl | H | Smp. 101-102° |
| 13.44 | H | Br | H | |
| 13.45 | F | Cl | $OC_2H_5$ | |
| 13.46 | F | Cl | $OC_3H_7$-n | |
| 13.47 | F | Cl | $OC_4H_9$-n | |
| 13.48 | F | Cl | $OCH(CH_3)C_2H_5$ | |
| 13.49 | F | Cl | $OCH_2CH(CH_3)_2$ | |
| 13.50 | F | Cl | $OCH_2C{\equiv}CH$ | $n_D^{22} = 1,5363$ |
| 13.51 | F | Cl | $OCH(CH_3)COOCH_3$ | |

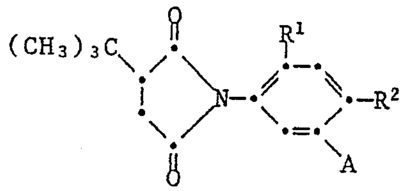

Tabelle 14

| Nr. | $R^1$ | $R^2$ | A |
|---|---|---|---|
| 14.01 | F | Cl | $COCH_3$ |
| 14.02 | F | Cl | $COCH_2Cl$ |
| 14.03 | F | Cl | $COCF_3$ |
| 14.04 | F | | $COCH_3$ |
| 14.05 | F | Cl | $C(CH_3)=N-OH$ |
| 14.06 | F | Cl | $C(CH_3)=N-OCH_3$ |
| 14.07 | F | Cl | $C(CN)=N-OH$ |
| 14.08 | F | Br | $C(CN)=N-OC_2H_5$ |
| 14.09 | F | Cl | $C(CN)=NOCH(CH_3)COOCH_3$ |
| 14.10 | F | Cl | 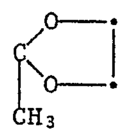 |
| 14.11 | F | Cl | 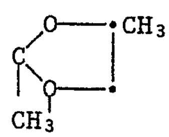 |
| 14.12 | F | Cl | 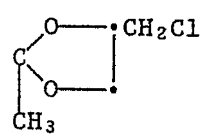 |
| 14.13 | F | Cl | COOH |
| 14.14 | F | Br | COOH |
| 14.15 | F | Cl | $COOCH_3$ |
| 14.16 | F | Cl | $COOCH(CH_3)_2$ |
| 14.17 | H | Cl | COOH |
| 14.18 | H | Cl | $COOCH_3$ |
| 14.19 | H | Cl | $COOCH(CH_3)_2$ |
| 14.20 | F | Cl | $COSCH_2COOCH_3$ |
| 14.21 | F | Cl | $COSCH_2COOCH_2(CH_3)_2$ |
| 14.22 | F | Cl | $COSCH_2(CH_3)COOCH_3$ |

Tabelle 14 (Fortsetzung)

| Nr. | $R^1$ | $R^2$ | A | |
|---|---|---|---|---|
| 14.23 | F | Cl | $COSCH(CH_3)COOCH_2CH=CH_2$ | |
| 14.24 | F | Cl | OH | |
| 14.25 | F | Br | OH | |
| 14.26 | F | Cl | $OCH_3$ | |
| 14.27 | F | Cl | $OCH(CH_3)_2$ | $n_D^{22} = 1.5185$ |
| 14.28 | F | Br | $OCH_2CH=CH_2$ | |
| 14.29 | F | Cl | $OCH_2CH=CHCl$ | |
| 14.30 | F | Cl | $OCH_2CCl=CH_2$ | |
| 14.31 | F | Cl | $OCH_2COOCH_3$ | |
| 14.32 | F | Cl | $OCH_2COOCH(CH_3)_2$ | |
| 14.33 | F | Cl | SH | |
| 14.34 | F | Br | SH | |
| 14.35 | F | Cl | $SCH_3$ | |
| 14.36 | F | Cl | $SCH(CH_3)_2$ | |
| 14.37 | F | Cl | $SCH_2CH=CHCl$ | |
| 14.38 | F | Cl | $SCH_2COOH$ | |
| 14.39 | F | Br | $SCH_2COOH$ | |
| 14.40 | F | Cl | $SCH(CH_3)COOH$ | |
| 14.41 | F | Cl | $SCH_2COOCH_3$ | |
| 14.42 | F | Cl | $SCH(CH_3)COOCH_3$ | |
| 14.43 | H | Cl | H | Smp. 195-196° |
| 14.44 | F | Cl | $OCH_2C{\equiv}CH$ | |
| 14.45 | F | Cl | $OCH(CH_3)COOCH_3$ | |

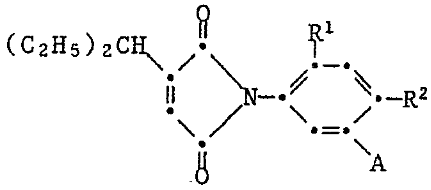

Tabelle 15

| Nr. | $R^1$ | $R^2$ | A |
|---|---|---|---|
| 15.01 | F | Cl | $COCH_3$ |
| 15.02 | F | Cl | $COCH_2Cl$ |
| 15.03 | F | Cl | $COCF_3$ |
| 15.04 | F | | $COCH_3$ |
| 15.05 | F | Cl | $C(CH_3)=N-OH$ |
| 15.06 | F | Cl | $C(CH_3)=N-OCH_3$ |
| 15.07 | F | Cl | $C(CN)=N-OH$ |
| 15.08 | F | Br | $C(CN)=N-OC_2H_5$ |
| 15.09 | F | Cl | $C(CN)=NOCH(CH_3)COOCH_3$ |
| 15.10 | F | Cl | 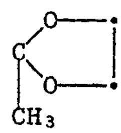 |
| 15.11 | F | Cl | 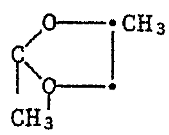 |
| 15.12 | F | Cl | 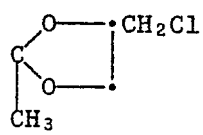 |
| 15.13 | F | Cl | COOH |
| 15.14 | F | Br | COOH |
| 15.15 | F | Cl | $COOCH_3$ |
| 15.16 | F | Cl | $COOCH(CH_3)_2$ |
| 15.17 | H | Cl | COOH |
| 15.18 | H | Cl | $COOCH_3$ |
| 15.19 | H | Cl | $COOCH(CH_3)_2$ |
| 15.20 | F | Cl | $COSCH_2COOCH_3$ |
| 15.21 | F | Cl | $COSCH_2COOCH_2(CH_3)_2$ |
| 15.22 | F | Cl | $COSCH_2(CH_3)COOCH_3$ |

Tabelle 15 (Fortsetzung)

| Nr. | $R^1$ | $R^2$ | A |
|---|---|---|---|
| 15.23 | F | Cl | $COSCH(CH_3)COOCH_2CH{=}CH_2$ |
| 15.24 | F | Cl | OH |
| 15.25 | F | Br | OH |
| 15.26 | F | Cl | $OCH_3$ |
| 15.27 | F | Cl | $OCH(CH_3)_2$ |
| 15.28 | F | Br | $OCH_2CH{=}CH_2$ |
| 15.29 | F | Cl | $OCH_2CH{=}CHCl$ |
| 15.30 | F | Cl | $OCH_2CCl{=}CH_2$ |
| 15.31 | F | Cl | $OCH_2COOCH_3$ |
| 15.32 | F | Cl | $OCH_2COOCH(CH_3)_2$ |
| 15.33 | F | Cl | SH |
| 15.34 | F | Br | SH |
| 15.35 | F | Cl | $SCH_3$ |
| 15.36 | F | Cl | $SCH(CH_3)_2$ |
| 15.37 | F | Cl | $SCH_2CH{=}CHCl$ |
| 15.38 | F | Cl | $SCH_2COOH$ |
| 15.39 | F | Br | $SCH_2COOH$ |
| 15.40 | F | Cl | $SCH(CH_3)COOH$ |
| 15.41 | F | Cl | $SCH_2COOCH_3$ |
| 15.42 | F | Cl | $SCH(CH_3)COOCH_3$ |
| 15.43 | F | Cl | $OCH_2C{\equiv}CH$ |
| 15.44 | F | Cl | $OCH(CH_3)COOCH_3$ |

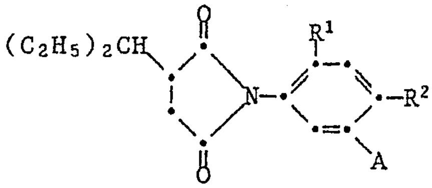

Tabelle 16

| Nr. | $R^1$ | $R^2$ | A |
|---|---|---|---|
| 16.01 | F | Cl | $COCH_3$ |
| 16.02 | F | Cl | $COCH_2Cl$ |
| 16.03 | F | Cl | $COCF_3$ |
| 16.04 | F | | $COCH_3$ |
| 16.05 | F | Cl | $C(CH_3)=N-OH$ |
| 16.06 | F | Cl | $C(CH_3)=N-OCH_3$ |
| 16.07 | F | Cl | $C(CN)=N-OH$ |
| 16.08 | F | Br | $C(CN)=N-OC_2H_5$ |
| 16.09 | F | Cl | $C(CN)=NOCH(CH_3)COOCH_3$ |
| 16.10 | F | Cl | 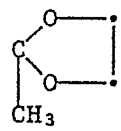 |
| 16.11 | F | Cl | 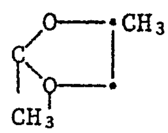 |
| 16.12 | F | Cl | 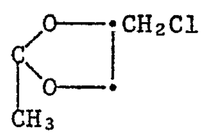 |
| 16.13 | F | Cl | COOH |
| 16.14 | F | Br | COOH |
| 16.15 | F | Cl | $COOCH_3$ |
| 16.16 | F | Cl | $COOCH(CH_3)_2$ |
| 16.17 | H | Cl | COOH |
| 16.18 | H | Cl | $COOCH_3$ |
| 16.19 | H | Cl | $COOCH(CH_3)_2$ |
| 16.20 | F | Cl | $COSCH_2COOCH_3$ |
| 16.21 | F | Cl | $COSCH_2COOCH_2(CH_3)_2$ |
| 16.22 | F | Cl | $COSCH_2(CH_3)COOCH_3$ |

Tabelle 16 (Fortsetzung)

| Nr. | $R^1$ | $R^2$ | A |
|---|---|---|---|
| 16.23 | F | Cl | $COSCH(CH_3)COOCH_2CH{=}CH_2$ |
| 16.24 | F | Cl | OH |
| 16.25 | F | Br | OH |
| 16.26 | F | Cl | $OCH_3$ |
| 16.27 | F | Cl | $OCH(CH_3)_2$ $n_D^{22} = 1.5229$ |
| 16.28 | F | Br | $OCH_2CH{=}CH_2$ |
| 16.29 | F | Cl | $OCH_2CH{=}CHCl$ |
| 16.30 | F | Cl | $OCH_2CCl{=}CH_2$ |
| 16.31 | F | Cl | $OCH_2COOCH_3$ |
| 16.32 | F | Cl | $OCH_2COOCH(CH_3)_2$ |
| 16.33 | F | Cl | SH |
| 16.34 | F | Br | SH |
| 16.35 | F | Cl | $SCH_3$ |
| 16.36 | F | Cl | $SCH(CH_3)_2$ |
| 16.37 | F | Cl | $SCH_2CH{=}CHCl$ |
| 16.38 | F | Cl | $SCH_2COOH$ |
| 16.39 | F | Br | $SCH_2COOH$ |
| 16.40 | F | Cl | $SCH(CH_3)COOH$ |
| 16.41 | F | Cl | $SCH_2COOCH_3$ |
| 16.42 | F | Cl | $SCH(CH_3)COOCH_3$ |
| 16.43 | F | Cl | $OCH_2C{\equiv}CH$ |
| 16.44 | F | Cl | $OCH(CH_3)COOCH_3$ |

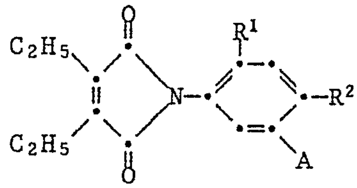

Tabelle 17

| Nr. | $R^1$ | $R^2$ | A |
|---|---|---|---|
| 17.01 | F | Cl | $COCH_3$ |
| 17.02 | F | Cl | $COCH_2Cl$ |
| 17.03 | F | Cl | $COCF_3$ |
| 17.04 | F | | $COCH_3$ |
| 17.05 | F | Cl | $C(CH_3)=N-OH$ |
| 17.06 | F | Cl | $C(CH_3)=N-OCH_3$ |
| 17.07 | F | Cl | $C(CN)=N-OH$ |
| 17.08 | F | Br | $C(CN)=N-OC_2H_5$ |
| 17.09 | F | Cl | $C(CN)=NOCH(CH_3)COOCH_3$ |
| 17.10 | F | Cl | 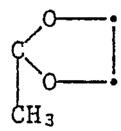 |
| 17.11 | F | Cl | 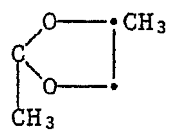 |
| 17.12 | F | Cl | 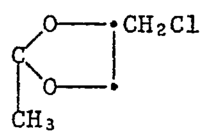 |
| 17.13 | F | Cl | COOH |
| 17.14 | F | Br | COOH |
| 17.15 | F | Cl | $COOCH_3$ |
| 17.16 | F | Cl | $COOCH(CH_3)_2$ |
| 17.17 | H | Cl | COOH |
| 17.18 | H | Cl | $COOCH_3$ |
| 17.19 | H | Cl | $COOCH(CH_3)_2$ |
| 17.20 | F | Cl | $COSCH_2COOCH_3$ |
| 17.21 | F | Cl | $COSCH_2COOCH_2(CH_3)_2$ |
| 17.22 | F | Cl | $COSCH_2(CH_3)COOCH_3$ |

Tabelle 17 (Fortsetzung)

| Nr. | $R^1$ | $R^2$ | A | |
|---|---|---|---|---|
| 17.23 | F | Cl | $COSCH(CH_3)COOCH_2CH=CH_2$ | |
| 17.24 | F | Cl | OH | |
| 17.25 | F | Br | OH | |
| 17.26 | F | Cl | $OCH_3$ | |
| 17.27 | F | Cl | $OCH(CH_3)_2$ | |
| 17.28 | F | Br | $OCH_2CH=CH_2$ | |
| 17.29 | F | Cl | $OCH_2CH=CHCl$ | |
| 17.30 | F | Cl | $OCH_2CCl=CH_2$ | |
| 17.31 | F | Cl | $OCH_2COOCH_3$ | |
| 17.32 | F | Cl | $OCH_2COOCH(CH_3)_2$ | |
| 17.33 | F | Cl | SH | |
| 17.34 | F | Br | SH | |
| 17.35 | F | Cl | $SCH_3$ | |
| 17.36 | F | Cl | $SCH(CH_3)_2$ | |
| 17.37 | F | Cl | $SCH_2CH=CHCl$ | |
| 17.38 | F | Cl | $SCH_2COOH$ | |
| 17.39 | F | Br | $SCH_2COOH$ | |
| 17.40 | F | Cl | $SCH(CH_3)COOH$ | |
| 17.41 | F | Cl | $SCH_2COOCH_3$ | |
| 17.42 | F | Cl | $SCH(CH_3)COOCH_3$ | |
| 17.43 | F | Cl | H | Oel |
| 17.44 | F | Cl | $OCH_2C{\equiv}CH$ | |
| 17.45 | F | Cl | $OCH(CH_3)COOCH_3$ | |

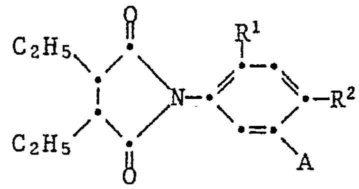

Tabelle 18

| Nr. | $R^1$ | $R^2$ | A |
|---|---|---|---|
| 18.01 | F | Cl | $COCH_3$ |
| 18.02 | F | Cl | $COCH_2Cl$ |
| 18.03 | F | Cl | $COCF_3$ |
| 18.04 | F | | $COCH_3$ |
| 18.05 | F | Cl | $C(CH_3)=N-OH$ |
| 18.06 | F | Cl | $C(CH_3)=N-OCH_3$ |
| 18.07 | F | Cl | $C(CN)=N-OH$ |
| 18.08 | F | Br | $C(CN)=N-OC_2H_5$ |
| 18.09 | F | Cl | $C(CN)=NOCH(CH_3)COOCH_3$ |
| 18.10 | F | Cl | 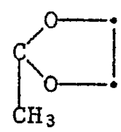 |
| 18.11 | F | Cl | 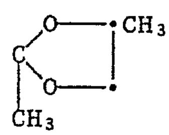 |
| 18.12 | F | Cl | 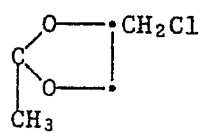 |
| 18.13 | F | Cl | COOH |
| 18.14 | F | Br | COOH |
| 18.15 | F | Cl | $COOCH_3$ |
| 18.16 | F | Cl | $COOCH(CH_3)_2$ |
| 18.17 | H | Cl | COOH |
| 18.18 | H | Cl | $COOCH_3$ |
| 18.19 | H | Cl | $COOCH(CH_3)_2$ |
| 18.20 | F | Cl | $COSCH_2COOCH_3$ |
| 18.21 | F | Cl | $COSCH_2COOCH_2(CH_3)_2$ |
| 18.22 | F | Cl | $COSCH_2(CH_3)COOCH_3$ |

Tabelle 18 (Fortsetzung)

| Nr. | $R^1$ | $R^2$ | A | |
|---|---|---|---|---|
| 18.23 | F | Cl | $COSCH(CH_3)COOCH_2CH=CH_2$ | |
| 18.24 | F | Cl | OH | |
| 18.25 | F | Br | OH | |
| 18.26 | F | Cl | $OCH_3$ | |
| 18.27 | F | Cl | $OCH(CH_3)_2$ | Smp. 80-81° |
| 18.28 | F | Br | $OCH_2CH=CH_2$ | |
| 18.29 | F | Cl | $OCH_2CH=CHCl$ | |
| 18.30 | F | Cl | $OCH_2CCl=CH_2$ | |
| 18.31 | F | Cl | $OCH_2COOCH_3$ | |
| 18.32 | F | Cl | $OCH_2COOCH(CH_3)_2$ | |
| 18.33 | F | Cl | SH | |
| 18.34 | F | Br | SH | |
| 18.35 | F | Cl | $SCH_3$ | |
| 18.36 | F | Cl | $SCH(CH_3)_2$ | |
| 18.37 | F | Cl | $SCH_2CH=CHCl$ | |
| 18.38 | F | Cl | $SCH_2COOH$ | |
| 18.39 | F | Br | $SCH_2COOH$ | |
| 18.40 | F | Cl | $SCH(CH_3)COOH$ | |
| 18.41 | F | Cl | $SCH_2COOCH_3$ | |
| 18.42 | F | Cl | $SCH(CH_3)COOCH_3$ | |
| 18.43 | F | Cl | H | Smp. 89-93° |
| 18.44 | F | Cl | $OCH_2C\equiv CH$ | |
| 18.45 | F | Cl | $OCH(CH_3)COOCH_3$ | |

Formulierungsbeispiele

Beispiel 15: Formulierungsbeispiele für flüssige Wirkstoffe der Formel I (% = Gewichtsprozent)

| a) Emulsionskonzentrate | a) | b) | c) |
|---|---|---|---|
| Wirkstoff gemäss Tabellen 1-10 | 20 % | 40 % | 50 % |
| Ca-Dodecylbenzolsulfonat | 5 % | 8 % | 5,8 % |
| Ricinusöl-polyäthylenglykoläther (36 Mol AeO) | 5 % | - | - |
| Tributylphenoyl-polyäthylenglykoläther (30 mol AeO) | - | 12 % | 4,2 % |
| Cyclohexanon | - | 15 % | 20 % |
| Xylolgemisch | 70 % | 25 % | 20 % |

Aus solchen Konzentraten können durch Verdünnen mit Wasser Emulsionen jeder gewünschten Konzentration hergestellt werden.

| b) Lösungen | a) | b) | c) | d) |
|---|---|---|---|---|
| Wirkstoff gemäss Tabellen 1-10 | 80 % | 10 % | 5 % | 95 % |
| Aethylenglykol-monomethyläther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 94 % | -ż |

Die Lösungen sind zur Anwendung in form kleinster Tropfen geeignet.

| c) Granulate | a) | b) |
|---|---|---|
| Wirkstoff gemäss Tabellen 1-10 | 5 % | 10 % |
| Kaolin | 94 % | - |
| Hochdisperse Kieselsäure | 1 % | |
| Attapulgit | - | 90 % |

Der Wirkstoff wird in Methylenchlorid gelöst, auf den Träger aufgesprüht und das Lösungsmittel anschliessend im Vakuum abgedampft.

| d) Stäubemittel | a) | b) |
|---|---|---|
| Wirkstoff gemäss Tabellen 1-10 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertiges Stäubemdittel.

Beispiel 16: Formulierungsbeispiele für feste Wirkstoffe der Formel I (% = Gewichtsprozent)

| a) Spritzpulver | a) | b) |
|---|---|---|
| Wirkstoff gemäss Tabellen 1-10 | 20 % | 60 % |
| Na-Ligninsulfonat | 5 % | 5 % |
| Na-Laurylsulfat | - | 6 % |
| Octylphenolpolyäthylenglykoläther (7-8 Mol Ae) | - | 2 % |
| Hochdisperse Kieselsäure | 5 % | 27 % |
| Kaolin | 67 % | - |

Der Wirkstoff wird mit den Zusatzstoffen gut vermischt und in einer geeigneten Mühle gut vermahlen. Man erhält Spritzpulver, die sich mit Wasser zur Suspension jeder gewünschten Konzentration verdünnen lassen.

b) Emulsions-Konzentrat

Wirkstoff gemäss Tabellen 1-10 10 %
Octylphenolpolyäthylenglykoläther
(4-5 Mol AeO) 3 %
Ca-Dodecylbenzolsulfonat 3 %
Ricinusölpolyglykoläther (36 Mol AeO) 4 %
Cyclohexanon 30 %
Xylolgemisch 50 %

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff gemäss Tabellen 1-10 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

d) Extruder Granulat

Wirkstoff gemäss Tabellen 1-10 10 %
Na-Ligninsulfonat 2 %
Carboxymethylcellulose 1 %
Kaolin 87 %

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

Wirkstoff gemäss Tabellen 1-10 3 %
Polyäthylenglykol (MG 200) 3 %
Kaolin 94 %

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

Wirkstoff gemäss Tabellen 1-10 40 %
Aethylenglykol 10 %
Nonylphenolpolyäthylenglykoläther (15 Mol AeO) 6 %
Na-Ligninsulfonat 10 %
Carboxymethylcellulose 1 %
37%ige wässrige Formaldehyd-Lösung 0,2 %
Silikonöl in Form einer 75%igen wässrigen Emulsion 0,8 %
Wasser 32 %

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

Beispiel 17: Pre-emergente Herbizid-Wirkung

Im Gewächshaus wird unmittelbar nach der Einsaat der Versuchspflanzen in Saatschalen die Erdoberfläche mit einer wässrigen Dispersion der Wirkstoffe, erhalten aus einem 25%igen Emulsionskonzentrat behandelt. Es werden Konzentrationen von 4 kg Wirksubstanz/Hektar angewendet. Die Saatschalen werden im Gewächshaus bei 22-25° C und 50-70 % relativer Luftfeuchtigkeit gehalten und der Versuch nach 3 Wochen ausgewertet und der Zustand der Pflanzen gemäss folgender Skala bewertet.

1 Pflanze abgestorben oder hat nicht gekeimt
2-4 starke Schädigung
5 mittlerer Schaden, Pflanze bleibt verkümmert
6-8 leichte Schäden
9 Pflanze gedeiht normal, wie unbehandelte Kontrollfplanzen.

Die Resultate dieses Versuches sind in der untenstehenden Tabelle festgehalten:

| Verbindung | 1.27 | | | | 1.43 | | | |
|---|---|---|---|---|---|---|---|---|
| Aufwandmenge g/ha | 1000 | 500 | 250 | 125 | 1000 | 500 | 250 | 125 |
| Pflanze | | | | | | | | |
| Gerste | 6 | 8 | 9 | 9 | 3 | 4 | 8 | 9 |
| Weizen | 8 | 9 | 9 | 9 | 7 | 8 | 9 | 9 |
| Mais | 7 | 7 | 9 | 9 | 5 | 8 | 9 | 9 |
| Reis | 4 | 6 | 8 | 9 | 7 | 8 | 9 | 9 |
| Avena fatua | 3 | 6 | 8 | 9 | 1 | 2 | 3 | 7 |
| Bromus tectorum | 4 | 5 | 7 | 9 | 2 | 4 | 4 | 7 |
| Lolium perenne | 2 | 3 | 4 | 7 | 1 | 1 | 1 | 3 |
| Alopecurus myosuroides | 1 | 2 | 4 | 5 | 1 | 2 | 4 | 4 |
| Digitaria sanguinalis | 1 | 1 | 1 | 1 | 1 | 1 | 1 | 1 |
| Echinochloa crus galli | 1 | 1 | 1 | 5 | 1 | 2 | 2 | 4 |
| Sorghum halepense | 1 | 1 | 1 | 7 | 1 | 2 | 5 | 6 |
| Rottboellia exaltata | 1 | 1 | 3 | 7 | 1 | 1 | 2 | 3 |
| Soja | 7 | 8 | 9 | 9 | 8 | 9 | 9 | 9 |
| Baumwolle | 7 | 8 | 9 | 9 | 4 | 9 | 9 | 9 |
| Sonnenblume | 8 | 9 | 9 | 9 | 7 | 8 | 9 | 9 |
| Abutilon | 1 | 1 | 1 | 3 | 1 | 1 | 2 | 4 |
| Sida spinosa | 1 | 2 | 2 | 2 | 1 | 1 | 2 | 2 |
| Amaranthus retroflexus | 1 | 1 | 1 | 1 | 1 | 1 | 1 | 1 |
| Chenopodium Sp. | 1 | 1 | 1 | 1 | 1 | 1 | 1 | 1 |
| Solanum nigrum | 1 | 1 | 1 | 2 | 1 | 1 | 1 | 1 |
| Stellaria media | 1 | 4 | 6 | 8 | 1 | 1 | 1 | 2 |
| Chrysanthemum leucum | 1 | 1 | 4 | 6 | 1 | 2 | 2 | 4 |
| Galium apanine | 2 | 2 | 2 | 6 | 2 | 3 | 3 | 7 |
| Viola tricolor | 1 | 1 | 1 | 1 | 1 | 1 | 1 | 1 |
| Veronica Sp. | 1 | 1 | 1 | 1 | 1 | 1 | 1 | 1 |

Beispiel 18: Post-emergente Herbizid-Wirkung (Kontaktherbizid)

Eine Anzahl Unkräuter, sowohl monokotyle wie dikotyle, wurden nach dem Auflaufen (im 4- bis 6-Blattstadium) mit einer wässrigen Wirkstoffdispersion in einer Dosierung von 4 kg Wirksubstanz pro Hektar auf die Pflanzen gespritzt und diese bei 24°-26°C und 45-60 % relativer Luftfeuchtigkeit gehalten. 15 Tage nach der Behandlung wird der Versuch nach der obigen Notenskala ausgewertet. Auch in diesem Versuch zeigen die Verbindungen der Tabellen 1 bis 18 starke bis sehr starke Herbizidwirkung.

Beispiel 19: Herbizidwirkung vor dem Auflaufen der Pflanzen

Kunststofföpfe werden mit expandiertem Vermiculit (Dichte: 0,135 g/cm³, Wasserabsorptionsvermögen: 0,565 l/l) gefüllt. Nach dem Sättigen des nicht adsorptiven Vermiculits mit einer wässrigen Wirkstoffemulsion in deiononisiertem Wasser, die die Wirkstoffe in einer Konzentration von 70,8 ppm enthält, werden Samen der folgenden Pflanzen auf die Oberfläche gesät. Nasturtium offcinalis, Agrostis tenuis, Stellaria media und Digitaria sanguinalis. Die Versuchsgefässe werden anschliessend in einer Klimakammer bei 20°C, einer Beleuchtung von ca. 20 kLux und einer relativen Luftfeuchtigkeit von 70 % gehalten. Während der Keimphase von 4 bis 6 Tagen werden die Töpfe zur Erhöhung der örtlichen Luftfeuchtigkeit mit lichtdurchlässigem Material abgedeckt und mit deinoisiertem Wasser begossen. Nach dem 5. Tag wird dem Giesswasser 0,5 % eines handelsüblichen Flüssigdüngers (®Greenzit) zugesetzt. 12 Tage nach der Aussaat wird der Versuch ausgewertet und der Zustand der Versuchspflanzen gemäss der Skala vom Beispiel 17 bewertet.

Die Resultate sind in der untenstehenden Tabelle zusammengefasst.

| Verbindung Nr. | Aufwand-menge | Nasturtium | Agrostis | Stellaria | Digitaria |
|---|---|---|---|---|---|
| 1.27 | 100 ppm | 1 | 1 | 2 | 1 |
| | 10 ppm | 2 | 1 | 2 | 2 |
| 9.27 | 100 ppm | 1 | 1 | 1 | 1 |
| | 10 ppm | 1 | 1 | 2 | 1 |
| 11.27 | 100 ppm | 2 | 2 | 2 | 2 |

Beispiel 20: Herbizidwirkung für Wasserreis (paddy)

Die Wasserunkräuter Echinochloa crus galli und Monocharia vag. werden in Plastikbechern (60 cm² Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat mit einer wässrigen Emulsion der Prüfsubstanzen durch eine Spritzung auf die Gefässe. Die daher verwendete Dosis entspricht einer Wirkstoffmenge von 0,5 bis 4 kg AS pro Hektar (Spritzbrühmenge = 550 l/ha. Die Pflanzenbecher werden dann im Gewächshaus unter optimalen Wachstumsbedingungen für die Reisunkräuter aufgestellt, d.h. bei 25°-30°C und hoher Luftfeuchtigkeit. Die Auswertung der Versuche findet 3 Wochen nach Applikation statt. Verbindungen der Tabellen 1 bis 18 schädigen dabei die Unkräuter, nicht aber den Reis.

Beispiel 21: Wuchshemmung bei tropischen Bodenbedecker-Leguminosen (cover crops)

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 6000 lux Kunstlicht, pro Tag 14 Stunden, bei Temperaturen von 27° bei Tag und 21°C bei Nacht gehalten. 4 Wochen nach der Applikation wird der Versuch ausge wertet. Es werden dabei der Neuzuwachs im Vergleich zur Kontrolle abgeschätzt und gewogen und die Phytotoxizität bewertet. In diesem Versuch zeigen die mit Wirkstoffen der Tabellen 1 bis 18 in Aufwandmengen von 50-3000 g/ha behandelten Pflanzen eine deutliche Reduktion des Neuzuwachses (weniger als 20 % des Neuzuwachses bei unbehandelten Kontrollpflanzen), ohne dass dabei die Versuchspflanzen geschädigt wurden.

Beispiel 22: Wuchsregulierung an Sojabohnen

In Kunststoffbehältern mit einem Erde-Torf-Sandgemisch im Verhältnis 6:3:1 werden Sojabohnen der Sorte "Hark" angesät und in eine Klimakammer gegeben. Durch optimale Tempeaturwahl, Beleuchtung, Düngerzugabe und Bewässerung entwickeln sich die Pflanzen nach ca. 5 Wochen bis zum 5-6 Trifolia-Blattstadium. Zu diesem Zeitpunkt werden die Pflanzen mit der wässrigen Brühe eines Wirkstoffs der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die geprüften erfindungsgemässen Wirkstoffe der Tabellen 1 bis 18 eine merklicher

Erhöhung der Anzahl und des Gewichts der Schoten im Haupttrieb.

Beispiel 23: Wuchshemmung bei Getreide

Im Kunststofftöpfen mit sterilisierter Erde werden in Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes besprüht. Die Wirkstoffmenge beträgt bis zu 100 g Aktivsubstanz pro Hektar. 21 Tage nach Applikation wird das Wachstum des Getreides beurteilt. Die mit erfindungsgemässen Wirkstoffen behandelten Pflanzen weisen im Vergleich zu unbehandelten Kontrollen eine Verringerung des Neuzuwachses (60-90 % der Kontrolle), sowie teilweise eine Zunahme der Stengeldurchmesser auf.

Beispiel 24: Wuchshemmung bei Gräsern

In Kunststoffschalen mit Erde-Torf-Sand-Gemisch (6:3:1) werden die Gräser Lolium perenne, Poa pratensis, Festuca ovina, Dactylis glomerata und Cynodon dactylon im Gewächshaus angesät und nach Bedarf bewässert. Die aufgeleufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes besprüht. Die Wirkstoffmenge beträgt umgerechnet bis zu 500 g Aktivsubstanz pro Hektar. 21 Tage nach Applikation wird das Wachstum der Gräser beurteilt.

Geprüfte Verbindungen der Tabellen 1 bis 18 bewirken eine Reduzierung des Neuzuwachses von um 10-30 % im Vergleich zur unbehandelten Kontrolle.

Biepiel 25: Desiccations- und Defoliationswirkung

In einem Gewächshaus wurden Baumwollpflanzen der Sorte Deltapine in Tontöpfen angezogen. Nach abgeschlossener Kapselbildung wurden sie mit wässerigen Zubereitungen eines Wirkstoffes in einer Aufwandmenge, die 1,2, 0,6 und 0,3 kg/ha im Feld entspräche, gespritzt. Unbehandelte Pflanzen wurden als Kontrolle belassen. Die Auswertung des Versuches erfolgte 3, 7 und 14 Tage nach Applikation der Wirksubstanz durch Bestimmen des Grades an Defoliation (% abgefallene Blätter) und an Desiccation (% Austrocknung der an der Pflanze verbleibenden Blätter).

In diesem Versuch verblieben auf den mit Verbindungen der Tabellen 1-18 bei Aufwandmengen von 0,6 und 1,2 kg/ha gespritzten Pflanzen nach 7 Tagen bloss noch wenige vertrocknete Blätter auf den Pflanzen (> 80 % Blattfall und Austrocknung).

**Patentansprüche**

1. N-Phenyl-maleinsäureimide und N-Phenylsuccinimide der Formel I

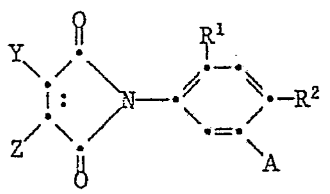

(I)

worin | : für eine Einfach- oder Doppelbindung,
$R^1$ für Wasserstoff oder Fluor,
$R^2$ für Halogen,
Y für $C_1$-$C_8$-Alkyl,
Z für Wasserstoff oder $C_1$-$C_8$-Alkyl, und
A für Wasserstoff oder eine Gruppe

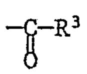 , 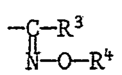 , 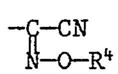 , 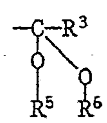 , 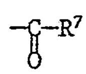 oder $-X-R^8$ stehen,

stehen,
wobei
$R^3$ Wasserstoff, $C_1$-$C_4$-Alkyl oder $C_1$-$C_4$-Halogenalkyl,
$R^4$ Wasserstoff, $C_1$-$C_8$-Alkyl, $C_2$-$C_8$-Alkoxyalkyl, $C_2$-$C_8$-Alkylthioalkyl, $C_2$-$C_8$-Alkylaminoalkyl, $C_1$-$C_8$-Halogenalkyl, $C_1$-$C_8$-Cyanoalkyl, $C_3$-$C_8$-Alkenyl, $C_3$-$C_8$-Halogenalkenyl, $C_3$-$C_8$-Alkinyl, $C_3$-$C_7$-Cycloalkyl, $C_3$-$C_7$-Halogencycloalkyl, $C_1$-$C_4$-Alkylsulfonyl, ein Natriumion, ein Kaliumion, ein Magnesiumion, ein Calciumion, ein Ammoniumion, $C_1$-$C_4$-Alkoxycarbonyl, Benzyloxycarbonyl, $C_5$-$C_7$-Cycloalkoxycarbonyl, Phenoxycarbonyl, das unsubstituiert oder am Phenylring einfach durch Halogen, $C_1$-$C_4$-Alkyl,

$C_1$-$C_4$-Alkoxy, $C_1$-$C_4$-Halogenalkyl, Cyan oder Nitro substituiert ist, $C_1$-$C_4$-Alkylthiocarbonyl, $C_1$-$C_4$-Alkylaminocarbonyl, Dimethylaminocarbonyl, Benzylaminocarbonyl, $C_3$-$C_7$-Cycloalkylaminocarbonyl, Phenylaminocarbonyl, das unsubstituiert oder am Phenylkern einfach durch Halogen, $C_1$-$C_4$-Alkyl, $C_1$-$C_4$-Alkoxy, $C_1$-$C_4$-Halogenalkyl, Cyan oder Nitro substituiert ist, $C_1$-$C_8$-Alkylcarbonyl, Allylcarbonyl, Benzylcarbonyl, das unsubstituiert oder am Phenylring einfach durch Halogen, $C_1$-$C_4$-Alkyl, $C_1$-$C_4$-Alkoxy, $C_1$-$C_4$-Halogenalkyl, Cyan oder Nitro substituiert ist, $C_3$-$C_7$-Cycloalkylcarbonyl, Benzoyl, das unsubstituiert oder am Phenylring einfach durch Halogen, $C_1$-$C_4$-Alkyl, $C_1$-$C_4$-Alkoxy, $C_1$-$C_4$-Halogenalkyl, Cyan oder Nitro substituiert ist, Furoyl, Thenoyl; oder $C_1$-$C_4$-Alkyl substituiert durch Phenyl, Halogenphenyl, $C_1$-$C_4$-Alkylphenyl, $C_1$-$C_4$-Alkoxyphenyl, $C_1$-$C_4$-Haloalkylphenyl, $C_1$-$C_4$-Halogenalkoxyphenyl, Nitrophenyl, Cyanophenyl, $C_1$-$C_8$-Alkoxycarbonyl, $C_2$-$C_8$-Alkoxyalkoxycarbonyl, $C_3$-$C_8$-Alkenyloxycarbonyl, $C_3$-$C_8$-Alkinyloxycarbonyl, $C_1$-$C_8$-Alkylthiocarbonyl, $C_3$-$C_8$-Alkenylthiocarbonyl, $C_3$-$C_8$-Alkinylthiocarbonyl, Carbamoyl, $C_1$-$C_4$-Alkylaminocarbonyl, Di-$C_1$-$C_4$-alkylaminocarbonyl, Phenylaminocarbonyl, das unsubstituiert oder am Phenylring einfach durch Halogen, $C_1$-$C_4$-Alkyl, $C_1$-$C_4$-Alkoxy, Cyan, Nitro oder $C_1$-$C_4$-Halogenalkyl substituiert ist, Dioxolan-2-yl, das unsubstituiert oder durch ein bis zwei $C_1$-$C_4$-Alkylreste substituiert ist, oder 1,3-Dioxan-2-yl, das unsubstituiert oder durch ein bis zwei $C_1$-$C_4$-Alkylreste substituiert ist;

$R^5$ und $R^6$ unabhängig voneinander jeweils $C_1$-$C_4$-Alkyl, $C_2$-$C_4$-Halogenalkyl oder $C_2$-$C_8$-Alkoxyalkyl, oder $R^5$ und $R^6$ zusammen eine Aethylen- oder Propylenbrücke oder einen 1,2-Cyclohexanylenkörper, wobei diese Gruppen unsubstituiert sind oder durch ein bis zwei Reste aus der Gruppe $C_1$-$C_4$-Alkyl, $C_1$-$C_4$-Halogenalkyl oder $C_1$-$C_4$-Hydroxyalkyl substituiert sind,

$R^7$ Hydroxy, $C_1$-$C_8$-Alkoxy, $C_2$-$C_8$-Alkoxyalkoxy, $C_2$-$C_8$-Alkylthioalkoxy, $C_2$-$C_8$-Alkylaminoalkoxy, $C_1$-$C_8$-Cyanoalkoxy, $C_3$-$C_8$-Alkenyloxy, $C_2$-$C_8$-Halogenalkenyloxy, $C_3$-$C_8$-Alkinyloxy, $C_3$-$C_7$-Cycloalkoxy, $C_3$-$C_7$-Halogencycloalkoxy, Benzloxy das unsubstituiert oder am Phenylkern einfach durch Halogen, $C_1$-$C_4$-Alkyl, $C_1$-$C_4$-Alkoxy, $C_1$-$C_4$-Halogenalkyl, Cyan oder Nitro substituiert ist, Phenoxy, Halogenphenoxy, $C_1$-$C_4$-Alkylphenoxy, $C_1$-$C_4$-Alkoxyphenoxy, $C_1$-$C_4$-Halogenalkylphenoxy, Cyanophenoxy, Nitrophenoxy, Phenylthio, Halogenphenylthio, $C_1$-$C_4$-Alkylphenylthio, $C_1$-$C_4$-Alkoxyphenylthio, $C_1$-$C_4$-Halogenalkylthio, Cyanophenylthio, Nitrophenylthio, die Salzgruppen -O-Na, -O-K, -O-Ca, -O-Mg, -O-$NH_4$; Amino, $C_1$-$C_4$-Alkylamino, Di-$C_1$-$C_4$-alkylamino, $C_2$-$C_4$-Halogenalkylamino, Di-$C_2$-$C_4$-halogenalkylamino, $C_1$-$C_4$-Hydroxyalkylamino, Di-$C_1$-$C_4$-hydroxyalkylamino, $C_3$-$C_4$-Alkenylamino, Diallylamino, -N-Pyrrolidino, -N-Piperidino, -N-Morpholino, -N-Thiomorpholino, -N-Piperidazino, $C_1$-$C_8$-Alkylthio, $C_3$-$C_8$-Alkenylthio, Benzylthio, $C_1$-$C_4$-Alkylthio substituiert durch $C_1$-$C_8$-Alkoxycarbonyl, $C_2$-$C_8$-Alkoxyalkoxycarbonyl, $C_3$-$C_8$-Alkenyloxycarbonyl, $C_3$-$C_8$-Alkinyloxycarbonyl, $C_1$-$C_8$-Alkylthiocarbonyl, $C_3$-$C_8$-Alkenylthiocarbonyl oder $C_3$-$C_8$-Alkinylthiocarbonyl; oder $C_1$-$C_4$-Alkoxy substituiert durch $C_1$-$C_8$-Alkoxycarbonyl, $C_2$-$C_8$-Alkoxyalkoxycarbonyl, $C_3$-$C_8$-Alkenyloxycarbonyl, $C_3$-$C_8$-Alkinyloxycarbonyl, $C_1$-$C_8$-Alkylthiocarbonyl, $C_3$-$C_8$-Alkenylthiocarbonyl, $C_3$-$C_8$-Alkinylthiocarbonyl, $C_1$-$C_4$-Alkylaminocarbonyl, Di-$C_1$-$C_4$-alkylaminocarbonyl oder Phenylaminocarbonyl das unsubstituiert oder am Phenylkern einfach durch Halogen, $C_1$-$C_4$-Alkyl, $C_1$-$C_4$-Alkoxy, $C_1$-$C_4$-Halogenalkyl, Cyan oder Nitro substituiert ist;

X Sauerstoff, Schwefel, -SO-, -$SO_2$-, -NH-, -N($C_1$-$C_4$-Alkyl)- oder -N($C_3$-$C_4$-Alkenyl)-, und

und

$R^8$ $C_1$-$C_8$-Alkyl, $C_2$-$C_8$-Alkoxyalkyl, $C_2$-$C_8$-Alkylthioalkyl, $C_2$-$C_8$-Alkylaminoalkyl, $C_2$-$C_8$-Halogenalkyl, $C_1$-$C_8$-Cyanoalkyl, $C_3$-$C_8$-Alkenyl, $C_2$-$C_8$-Halogenalkenyl, $C_3$-$C_8$-Alkinyl, $C_3$-$C_7$-Cycloalkyl, $C_3$-$C_7$-Halogencycloalkyl, $C_1$-$C_8$-Alkylcarbonyl, Allylcarbonyl, Benzylcarbonyl, das unsubstituiert oder am Phenylring einfach durch Halogen, $C_1$-$C_4$-Alkyl, $C_1$-$C_4$-Alkoxy, $C_1$-$C_4$-Halogenalkyl, Cyan oder Nitro substituiert ist, $C_3$-$C_7$-Cycloalkylcarbonyl, Benzoyl, das unsubstituiert oder am Phenylring einfach durch Halogen, $C_1$-$C_4$-Alkyl, $C_1$-$C_4$-Alkoxy, $C_1$-$C_4$-Halogenalkyl, Cyan oder Nitro substituiert ist, Furoyl, Thienoyl oder $C_1$-$C_4$-Alkyl substituiert durch Phenyl, Halogenphenyl, $C_1$-$C_4$-Alkylphenyl, $C_1$-$C_4$-Alkoxyphenyl, $C_1$-$C_4$-Haloalkylphenyl, $C_1$-$C_4$-Halogenalkoxyphenyl, Nitrophenyl, Cyanophenyl, $C_1$-$C_8$-Alkoxycarbonyl, $C_2$-$C_8$-Alkoxyalkoxycarbonyl, $C_3$-$C_8$-Alkenyloxycarbonyl, $C_3$-$C_8$-Alkinyloxycarbonyl, $C_1$-$C_8$-Alkylthiocarbonyl, $C_3$-$C_8$-Alkenylthiocarbonyl, $C_3$-$C_8$-Alkinylthiocarbonyl, Carbamoyl, $C_1$-$C_4$-Alkylaminocarbonyl, Di-$C_1$-$C_4$-alkylaminocarbonyl, Phenylaminocarbonyl, das unsubstitiert oder am Phenylring einfach durch Halogen, $C_1$-$C_4$-Alkyl, $C_1$-$C_4$-Alkoxy, Cyan, Nitro oder $C_1$-$C_4$-Halogenalkyl substituiert ist; Dioxolan-2-yl, das unsubstituiert oder durch ein bis zwei $C_1$-$C_4$-Alkylreste substituiert ist, oder 1,3-Dioxan-2-yl, das unsubstituiert oder durch ein bis zwei $C_1$-$C_4$-Alkylreste substituiert ist, bedeuten.

2. Verbindungen gemäss Anspruch 1, in denen

$R^1$ Wasserstoff oder Fluor,

$R^2$ Chlor oder Brom,

Y $C_1$-$C_8$-Alkyl,

Z Wasserstoff oder $C_1$-$C_8$-Alkyl,

A den Rest -$COR^3$ und

$R^3$ Wasserstoff, $C_1$-$C_4$-Alkyl oder $C_1$-$C_4$-Halogenalkyl bedeutet.

3. Verbindungen gemäss Anspruch 1, in denen

$R^1$ Wasserstoff oder Fluor,

$R^2$ Chlor oder Brom,

Y $C_1$-$C_8$-Alkyl,

Z Wasserstoff oder $C_1$-$C_8$-Alkyl

A Den Rest $-CR^3{=}NOR^4$ und
$R^3$ und $R^4$ die im Anspruch 1 gegebene Bedeutung haben.

4. Verbindungen gemäss Anspruch 1, in denen
$R^1$ Wasserstoff oder Fluor,
$R^2$ Chlor oder Brom,
Y $C_1$-$C_8$-Alkyl,
Z Wasserstoff oder $C_1$-$C_8$-Alkyl,
A den Rest $-C(CN){=}NOR^4$ bedeuten, und
$R^4$ die im Anspruch 1 gegebene Bedeutung hat.

5. Verbindungen gemäss Anspruch 1, in denen
$R^1$ Wasserstoff oder Fluor,
$R^2$ Chlor oder Brom,
Y $C_1$-$C_8$-Alkyl,
Z Wasserstoff oder $C_1$-$C_8$-Alkyl,
A den Rest

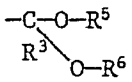

bedeuten und
$R^3$, $R^5$ und $R^6$ die im Anspruch 1 gegebene Bedeutung haben.

6. Verbindungen gemäss Anspruch 1, in denen
$R^1$ Wasserstoff oder Fluor,
$R^2$ Chlor oder Brom,
Y $C_1$-$C_8$-Alkyl,
Z Wasserstoff oder $C_1$-$C_8$-Alkyl,
A den Rest $-COR^7$ bedeuten, und
$R^7$ die im Anspruch 1 gegebene Bedeutung hat.

7. N-(2-Fluor-4-chlor-5-isopropoxyphenyl)-2,3-dimethyl-maleinsäureimid gemäss Anspruch 1.

8. N-(2-Fluor-4-chlor-5-isopropoxyphenyl)-2-n-butyl-3-methyl-maleinsäureimid gemäss Anspruch 1.

9. N-(2-Fluor-4-chlor-5-isopropoxycarbonylphenyl)-2-ethyl-3-methylmaleinsäureimid gemäss Anspruch 1.

10. N-(2-Fluor-4-chlor-5-isopropoxyphenyl)-2-ethyl-3-methylmaleinsäureimid gemäss Anspruch 1.

11. N-(2-Fluor-4-chlor-5-isopropoxycarbonylhpenyl)-2,3-dimethylmaleinsäureimid gemäss Anspruch 1.

12. Verbindungen gemäss Anspruch 1, in denen
$R^1$ Wasserstoff oder Fluor,
$R^2$ Chlor oder Brom,
Y und Z je Methyl bedeuten, und
A eine der im Anspruch 1 gegebenen Bedeutungen hat.

13. Verbindungen gemäss Anspruch 1, in denen
$R^1$ Wasserstoff oder Fluor,
$R^2$ Chlor oder Brom,
Y Aethyl,
Z Methyl bedeuten, und
A eine der im Anspruch 1 gegebenen Bedeutungen hat.

14. Verbindungen gemäss Anspruch 1, in denen
$R^1$ Wasserstoff oder Fluor,
$R^2$ Chlor oder Brom,
Y Isopropyl,
Z Methyl bedeuten und
A eine der im Anspruch 1 gegebenen Bedeutungen hat.

15. Verbindungen gemäss Anspruch 1, in denen
$R^1$ Wasserstoff oder Fluor,
$R^2$ Chlor oder Brom,
Y Isopropyl,
Z Wasserstoff bedeuten, und
A eine der im Anspruch 1 gegebenen Bedeutungen hat.

16. Verbindungen gemäss Anspruch 1, in denen
$R^1$ Wasserstoff oder Fluor,
$R^2$ Chlor oder Brom,
Y n-Butyl,
Z Methyl bedeuten, und
A eine der im Anspruch 1 gegebenen Bedeutungen hat.

17. Verfahren zur Herstellung der Verbindungen gemäss Anspruch 1, dadurch gekennzeichnet, dass man ein Maleinsäureanhydrid oder Bernsteinsäureanhydrid der Formel II

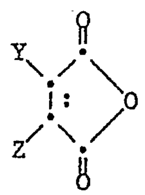

(II)

worin |: eine Einfach- oder Doppelbindung bedeutet und Y und Z die im Anspruch 1 gegebene Bedeutung haben, mit einem Anilin der Formel III

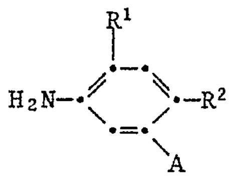

(III)

worin A, $R^1$ und $R^2$ die unter Formel I in Anspruch 1 gegebenen Bedeutungen haben, kondensiert.

18. Ein herbizides und pflanzenwachstumsregulierendes Mittel, dadurch gekennzeichnet, dass es neben Träger- und/oder anderen Zuschlagstoffen als Wirkstoff ein N-Phenylmaleinsäureimid oder N-Phenylsuccinimid der Formel I, Anspruch 1 enthält.

19. Die Verwendung eines Wirkstoffes gemäss Anspruch 1, oder eines einen solchen Wirkstoff enthaltenden Mittels zur Bekämpfung von Unkräutern.

20. Die Verwendung eines Wirkstoffes gemäss Anspruch 1 oder eines einen solchen Wirkstoff enthaltenden Mittels zur Hemmung des Pflanzenwachstums von Nutzpflanzen.

21. Ein Verfahren zur Bekämpfung von Unkräutern, dadurch gekennzeichnet, dass man die Unkräuter oder ihren Lebensraum mit einer wirksamen Menge eines Wirkstoffes der Formel I gemäss Anpsruch 1 behandelt.

22. Verfahren gemäss Anspruch 21, zur selektiven Unkrautbekämpfung.

23. Verfahren gemäss Anspruch 21, zur selektiven pre- oder postemergenten Bekämpfung von Unkräutern in Nutzpflanzenkulturen.

24. Verfahren gemäss Anspruch 21, in Nutzpflanzenkulturen wie Soja, Baumwolle, Hafer, Roggen, Hirse, Mais, Weizen, Gerste und Reis.

25. Anilinderivate der Formel III

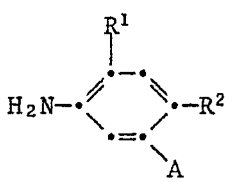

(III)

worin A, $R^1$ und $R^2$ die unter Formel I in Anspruch 1 gegebenen Bedeutungen haben als Zwischenprodukte zur Herstellung der N-Phenylmaleinsäureimide und N-Phenylsuccinimide gemäss Anspruch 1.

26. Säurechloride der Formel X

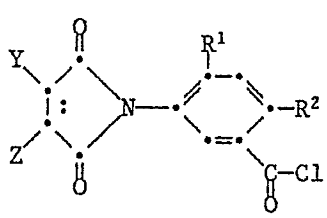

worin |: eine Einfach- oder Doppelbindung,
$R^1$ Wasserstoff oder Fluor,
$R^2$ Chlor oder Brom,
Y $C_1$-$C_8$-Alkyl und
Z Wasserstoff oder $C_1$-$C_8$-Alkyl bedeuten, als Zwischenprodukte zur Herstellung von N-Phenylmaleinsäureimiden oder N-Phenylsuccinimiden gemäss Anspruch 1.

27. 2-Nitro-5-malein- oder succinimido-benzoesäuren der Formel

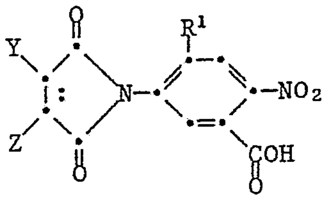

EMI PA=86 FR=3 HE=30 WI=45 TI=CHE

worin ⁞ : eine Einfach- oder Doppelbindung
$R^1$ Wasserstoff oder Fluor
Y $C_1$-$C_8$-Alkyl und
Z Wasserstoff oder $C_1$-$C_8$-Alkyl bedeuten, als Zwischenprodukte zur Herstellung von N-Phenylmaleinsäureimiden oder N-Phenylsuccinimiden gemäss Anspruch 1.

28. 2-Amino-5-malein- oder succinimidobenzoesäuren der Formel

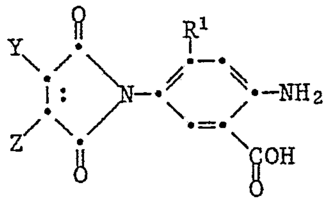

worin ⁞ : eine Einfach- oder Doppelbindung
$R^1$ Wasserstoff oder Fluor
Y $C_1$-$C_8$-Alkyl und
Z Wasserstoff oder $C_1$-$C_8$-Alkyl bedeuten, als Zwischenprodukt zur Herstellung von N-Phenylmaleinsäureimiden oder N-Phenylsuccinimiden gemäss Anspruch 1.